(19) Europäisches Patentamt
European Patent Office
Office européen des brevets

(11) EP 1 513 484 B1

(12) # EUROPÄISCHE PATENTSCHRIFT

(45) Veröffentlichungstag und Bekanntmachung des Hinweises auf die Patenterteilung:
**10.05.2006 Patentblatt 2006/19**

(21) Anmeldenummer: **02791751.7**

(22) Anmeldetag: **02.12.2002**

(51) Int Cl.:
***A61K 8/40*** (2006.01) ***A61Q 5/02*** (2006.01)

(86) Internationale Anmeldenummer:
**PCT/EP2002/013585**

(87) Internationale Veröffentlichungsnummer:
**WO 2003/047539 (12.06.2003 Gazette 2003/24)**

(54) **VERWENDUNG VON ECTOIN UND ECTOINDERIVATEN ZUR BEHANDLUNG VON HAAREN**
USE OF ECTOIN AND ECTOIN DERIVATIVES FOR THE TREATMENT OF HAIR
UTILISATION D'ECTOINE OU DE DERIVES D'ECTOINE POUR LE TRAITEMENT DES CHEVEUX

(84) Benannte Vertragsstaaten:
**AT BE BG CH CY CZ DE DK EE ES FI FR GB GR IE IT LI LU MC NL PT SE SI SK TR**

(30) Priorität: **05.12.2001 DE 10159781**

(43) Veröffentlichungstag der Anmeldung:
**16.03.2005 Patentblatt 2005/11**

(73) Patentinhaber: **Henkel Kommanditgesellschaft auf Aktien**
**40589 Düsseldorf (DE)**

(72) Erfinder:
- **KAINZ, Sabine**
  **47447 Moers (DE)**
- **NEUMANN, Frank**
  **40219 Düsseldorf (DE)**
- **POPPE, Elisabeth**
  **22763 Hamburg (DE)**
- **WESER, Gabriele**
  **25474 Bönningstedt (DE)**
- **HOLTMANN, Werner**
  **40724 Hilden (DE)**
- **FISCHER, Detlef**
  **41372 Niederkrüchten (DE)**
- **HEYER, Michael**
  **40699 Erkrath (DE)**
- **KALISCHKE, Swenja**
  **47509 Rheurdt (DE)**

(56) Entgegenhaltungen:
**DE-A- 10 002 725** **DE-A- 19 613 567**
**DE-A- 19 738 303** **DE-A- 19 933 463**

- **DATABASE WPI Section Ch, Week 199133 Derwent Publications Ltd., London, GB; Class A96, AN 1991-242247 XP002259676 & JP 03 157316 A (SUNSTAR KK), 5. Juli 1991 (1991-07-05) in der Anmeldung erwähnt**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 513 484 B1

## Beschreibung

Die Erfindung betrifft die Verwendung einer oder mehrerer Derivate heterocyclischer Carbonsäuren in kosmetischen Mitteln zur Reinigung und/oder zur pflegenden und regenerierenden Behandlung geschädigter Haare.

Gesunde und glänzende Haare in Verbindung mit einer dekorativen Gestaltung der Frisur stellen heutzutage wichtige Bestandteile der menschlichen Körperpflege dar. Um diese Ziele zu erreichen werden die Kopfhaare in vielfältiger Weise mit Reinigungs- und Pflege-, Glättungs- oder Well-, Festigungs- oder Verformungsmitteln, Färbe- oder Tönungsmitteln behandelt. Dies geschieht üblicherweise mit Zubereitungen auf wässriger Basis oder mit Zubereitungen, die nach der Behandlung mit Wasser ausgespült werden, und es ist daher prinzipiell im Anschluss an die Behandlung erforderlich, das Haar wieder vom Wasser zu befreien, oder es zu trocknen. Um dies in relativ kurzer Zeit zu erzielen, wird gewöhnlich die Trocknung der Haare unter Verwendung von Heißluft durchgeführt. Die Verwendung von Heißluftföns oder Trockenhauben dient aber auch einer Verstärkung der formgebenden und der festigenden Behandlung, beispielsweise durch Haarwickler oder festigende Polymere. Auch Lockenstäbe, die unter Hitze eine dauerhafte Fixierung oder Haarkräuselung bewirken sollen, finden eine breite Anwendung.

Durch die genannten Verfahren der Heißluft- und Hitzeanwendung zur Trocknung und Formgebung der Haare können diese jedoch, insbesondere bei unsachgemäßem Vorgehen, in ihrer Struktur geschädigt werden. Um diesem Problem entgegenzuwirken, wurden in der letzten Zeit zahlreiche Produkte entwickelt, die den hohen Anforderungen der Kosmetik genügen und gleichzeitig die thermische Haarschädigung verringern.

In JP 03157316 A2 wird z. B. vorgeschlagen, eine Kombination aus quartären Ammoniumsalzen, bestimmten Pflanzenextrakten und wasserlöslichen Polymeren mit quartären Ammoniumgruppen einzusetzen.

Gemäß WO 99/11224 eignet sich eine Kombination aus einem faserstrukturverbessernden Wirkstoff wie z. B. Panthenol und einem konditionierenden Wirkstoff wie z. B. einem kationischen Tensid, um die Hitzeschädigung des Haars zu vermindern.

Die Firma Beiersdorf offenbart in einer Reihe von Anmeldungen, beispielsweise in der DE 199 33 463, Hautpflegemittel auf der Basis von Ectoin und Ectoinderivaten sowie deren Verwendung, vorwiegend in Sonnenschutzformulierungen für die Haut. Die fakultative Verwendung von Ectoin oder Ectoinderivaten bei der Behandlung oxidativ geschädigter Haare wird ebenfalls genannt.

In der EP 671 161 A1 werden Ectoin und Ectoinderivate in kosmetischen Zubereitungen zur Behandlung von gealterter und gereizter Haut offenbart. Die WO 00/07560 beansprucht die Verwendung von Ectoin und bestimmter Derivate des Ectoins in kosmetischen Formulierungen zum Schutz der menschlichen Haut vor Trockenheit und zu hohen Salzkonzentrationen.

Auf der Suche nach wirksamen Zusätzen in Haarreinigungs- und Haarbehandlungsmitteln, die das Haar vor Hitzeschäden und vor Austrocknung schützen und gleichzeitig den Folgen dieser Schäden entgegenwirken, wurde überraschend gefunden, dass die Verbindungen der allgemeinen Formel (I) oder (II) diesen Anforderungen in hohem Maße gerecht werden.

Ein erster Gegenstand der vorliegenden Erfindung ist somit die Verwendung mindestens einer Verbindung der allgemeinen Formel (I) oder (II),

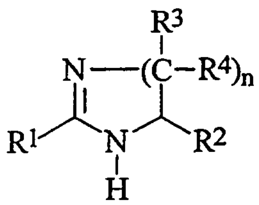

(I)

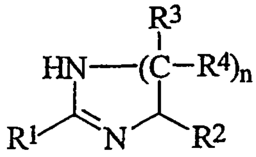

(II)

und/oder eines physiologisch verträglichen Salzes und/oder einer isomeren oder stereoisomeren Form dieser Verbindungen in Haarreinigungs- und/oder Haarbehandlungsmitteln zum Schutz der Haare vor Hitzeschäden, in denen

- $R^1$ steht für ein Wasserstoffatom, einen verzweigten oder unverzweigten $C_1$ - $C_4$-Alkylrest oder einen $C_2$ - $C_4$-Hydroxyalkylrest,
- $R^2$ steht für ein Wasserstoffatom, eine Gruppierung $-COOR^5$ oder eine Gruppierung $-CO(NH)R^5$, wobei $R^5$ für ein

Wasserstoffatom, einen $C_1$ - $C_4$-Alkylrest, einen Aminosäurerest, einen Dipeptid- oder einen Tripeptidrest stehen kann,

- $R^3$ und $R^4$ stehen unabhängig voneinander für ein Wasserstoffatom, einen $C_1$ - $C_4$-Alkylrest oder einer der beiden Reste steht für eine Hydroxygruppe und
- n steht für eine ganze Zahl von 1 bis 3.

Geeignete physiologisch verträgliche Salze der allgemeinen Verbindungen gemäß der Formel (I) oder (II) sind beispielsweise die Alkali-, Erdalkali-, Ammonium-, Triethylamin- oder Tris-(2-hydroxyethyl)aminsalze sowie solche, die sich aus der Umsetzung von Verbindungen gemäß der Formel (I) oder (II) mit anorganischen und organischen Säuren wie Salzsäure, Phosphorsäure, Schwefelsäure, verzweigten oder unverzweigten, substituierten oder unsubstituierten (beispielsweise durch eine oder mehrere Hydroxygruppen) $C_1$ - $C_4$-Mono- oder Dicarbonsäuren, aromatische Carbonsäuren und Sulfonsäuren wie Essigsäure, Citronensäure, Benzoesäure, Maleinsäure, Fumarsäure, Weinsäure und p-Toluolsulfonsäure ergeben. Beispiele für besonders bevorzugte physiologisch verträgliche Salze sind die Na-, K-, Mg- und Ca- und Ammoniumsalze der Verbindungen gemäß der Formel (I) oder (II), sowie die Salze, die sich durch Umsetzung von Verbindungen gemäß der Formel (I) oder (II) mit Salzsäure, Essigsäure, Citronensäure und Benzoesäure ergeben.

Unter isomeren oder stereoisomeren Formen der Verbindungen gemäß Formel (I) oder (II) werden erfindungsgemäß alle auftretenden optischen Isomere, Diastereomere, Racemate, Zwitterionen, Kationen oder Gemische davon verstanden.

Unter dem Begriff Aminosäure werden die stereoisomeren Formen, z.B. D- und L-Formen, folgender Verbindungen verstanden: Asparagin, Arginin, Asparaginsäure, Glutamin, Glutaminsäure, β-Alanin, γ-Aminobutyrat, $N_\varepsilon$-Acetyllysin, $N_\delta$-Acetylornitin, $N_\gamma$-Acetyldiaminobutyrat, $N_\alpha$-Acetyldiaminobutyrat, Histidin, Isoleucin, Leucin, Methionin, Phenylalanin, Serin, Threonin und Tyrosin.

L-Aminosäuren sind bevorzugt. Aminosäurereste leiten sich von den entsprechenden Aminosäuren ab. Die folgenden Aminosäurereste sind bevorzugt:

Gly, Ala, Ser, Thr, Val, β-Ala, γ-Aminobutyrat, Asp, Glu, Asn, Aln, $N_\varepsilon$-Acetyllysin, $N_\delta$-Acetylornithin, Ny-Acetyldiaminobutyrat, $N_\alpha$-Acetyldiaminobutyrat.

Die Kurzschreibweise der Aminosäuren erfolgte nach der allgemein üblichen Schreibweise. Die Di- oder Tripeptidreste sind in ihrer chemischen Natur nach Säureamide und zerfallen bei der Hydrolyse in 2 oder 3 Aminosäuren. Die Aminosäuren in dem Di- oder Tripeptidrest sind durch Amidbindungen miteinander verbunden.

Bezüglich der Herstellung der Di- und Tripeptidreste wird ausdrücklich auf die EP 0 671 161 A1 der Firma Marbert verwiesen. Auch Beispiele für Di- und Tripeptidreste sind der Offenbarung der EP 0 671 161 A1 zu entnehmen.

Beispiele für $C_1$ - $C_4$-Alkylgruppen in den erfindungsgemäßen Verbindungen sind Methyl, Ethyl, n-Propyl, Isopropyl, n-Butyl, Isobutyl und tert.-Butyl. Bevorzugte Alkylgruppen sind Methyl und Ethyl, Methyl ist eine besonders bevorzugte Alkylgruppe. Bevorzugte $C_2$ - $C_4$-Hydroxyalkylgruppen sind die Gruppen 2-Hydroxyethyl, 3-Hydroxypropyl oder 4-Hydroxybutyl; 2-Hydroxyethyl ist eine besonders bevorzugte Hydroxyalkylgruppe.

Ein zweiter Gegenstand der Erfindung ist die Verwendung mindestens einer Verbindung der allgemeinen Formel (I) oder (II) in Haarreinigungs- und/oder Haarbehandlungsmitteln zur Regulierung des Feuchtigkeitsgehalts der Haare.

Unter Regulierung des Feuchtigkeitsgehalts der Haare durch Verbindungen der allgemeinen Formel (I) oder (II) wird erfindungsgemäß sowohl der prophylaktische Schutz vor Austrocknung durch übermäßigen Wasserverlust unter Einwirkung von Hitze, als auch das erhöhte Wasseraufnahmepotential bereits geschädigter und ausgetrockneter Haare durch die Behandlung mit den erfindungsgemäßen Verbindungen nach Formel (I) oder (II) verstanden.

Ein dritter Gegenstand der Erfindung ist die Verwendung mindestens einer Verbindung der allgemeinen Formel (I) oder (II) in Haarreinigungs- und/oder Haarbehandlungsmitteln zur Steigerung der Widerstandskraft von Haaren.

Ein vierter Gegenstand der Erfindung ist die Verwendung mindestens einer Verbindung der allgemeinen Formel (I) oder (II) in Haarreinigungs- und/oder Haarbehandlungsmitteln zur Erhöhung des Haarglanzes.

Ein fünfter Gegenstand der Erfindung ist die Verwendung mindestens einer Verbindung der allgemeinen Formel (I) oder (II) in Haarreinigungs- und/oder Haarbehandlungsmitteln zur Verminderung von Haarbrüchen und Spliss.

Bevorzugte Verbindungen gemäß Formel (I) oder (II) sind solche, bei denen der Rest $R^1$ für eine Methylgruppe steht.

Weiterhin sind diejenigen Verbindungen gemäß der allgemeinen Formel (I) oder (II) begünstigt, bei denen der Rest $R^2$ für die Gruppierung -COOH steht

Bevorzugt sind auch Verbindungen der allgemeinen Formel (I) oder (II), bei denen die Reste $R^3$ und $R^4$ für Wasserstoff, oder jeweils unabhängig voneinander für Wasserstoff oder eine Hydroxygruppe stehen.

Ferner sind besonders solche Verbindungen gemäß der allgemeinen Formel (I) oder (II) bevorzugt, bei denen n für die Zahl 2 steht.

Eine ganz besonders bevorzugte Verbindung gemäß der allgemeinen Formel (I) oder (II) ist (S)-2-Methyl-1,4,5,6-tetrahydro-4-pyrimidincarbonsäure (Ectoin) sowie die physiologisch verträglichen Salze dieser Verbindung.

Eine weiterhin ganz besonders bevorzugte Verbindung gemäß der allgemeinen Formel (I) oder (II) ist (S,S)-5-Hydroxy-2-methyl-1,4,5,6-tetrahydro-4-pyrimidincarbonsäure (Hydroxyectoin) sowie die physiologisch verträglichen Salze dieser Verbindung.

Die Verbindungen der allgemeinen Formel (I) oder (II), insbesondere Ectoin, können erfindungsgemäß in allen Mitteln verwendet werden, die der Reinigung und Behandlung von Haaren dienen, beispielsweise in Haarshampoos, Haarspülungen, Haarkuren, Haar-Tonics, Dauerwell-Fixierlösungen, Haarfärbemitteln, Haarfärbeshampoos, Haarfestigern, Haarlegemitteln, Haarstyling-Zubereitungen, z.B. Fönwell-Lotionen, Schaumfestigern, Haargelen, Haarwachsen und anderen Haarreinigungs- und Haarbehandlungsmitteln. Insbesondere bevorzugt wird Ectoin oder ein Ectoinderivat in solchen Haarreinigungs- und Haarbehandlungsmitteln verwendet, nach deren Anwendung das Haar üblicherweise durch Anwendung von Heißluft getrocknet wird.

Generell können die Verbindungen gemäß der allgemeinen Formel (I) oder (II), insbesondere Ectoin, in den Haarreinigungs- und Haarbehandlungsmitteln in einer Konzentration von 0,01 - 2 Gew.-%, vorzugsweise 0,1 - 1 Gew.-%, jeweils bezogen auf das Gesamtgewicht des kosmetischen Mittels, enthalten sein.

Die kosmetischen Zubereitungen können neben den Verbindungen gemäß der allgemeinen Formel (I) oder (II) noch weitere Komponenten enthalten, die für den jeweiligen Anwendungszweck vorteilhaft und üblich sind: Haarreinigungsmittel (Shampoos) können beispielsweise schäumende anionische, zwitterionische, ampholytische und nichtionische Tenside enthalten. Haarspülmittel und Avivagemittel enthalten bevorzugt kationische Tenside und wasserlösliche Polymere mit quartären Ammoniumgruppen zur Verringerung der statischen Aufladbarkeit und zur Verbesserung der Kämmbarkeit. Dauerwell-Fixiermittel enthalten bevorzugt Oxidationsmittel wie z. B. Kaliumbromat oder Wasserstoffperoxid. Haarfärbeshampoos enthalten direktziehende Haarfärbemittel oder Oxidationsfarbstoff-Vorprodukte. Haarfestiger und Haarlegemittel sowie andere Haarstyling-Zubereitungen enthalten üblicherweise filmbildende in wässrigen oder wässrig-alkoholischen Medien lösliche Polymerisate, gegebenenfalls gemeinsam mit kationischen Tensiden oder kationischen Polymeren.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 8 bis 30 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 bis 4 C-Atomen in der Alkanolgruppe,

- lineare und verzweigte Fettsäuren mit 8 bis 30 C-Atomen (Seifen),
- Ethercarbonsäuren der Formel $R-O-(CH_2-CH_2O)_x-CH_2-COOH$, in der R eine lineare Alkylgruppe mit 8 bis 30 C-Atomen und x = 0 oder 1 bis 16 ist,
- Acylsarcoside mit 8 bis 24 C-Atomen in der Acylgruppe,
- Acyltauride mit 8 bis 24 C-Atomen in der Acylgruppe,
- Acylisethionate mit 8 bis 24 C-Atomen in der Acylgruppe,
- Sulfobemsteinsäuremono- und -dialkylester mit 8 bis 24 C-Atomen in der Alkylgruppe und Sulfobernsteinsäuremonoalkylpolyoxyethylester mit 8 bis 24 C-Atomen in der Alkylgruppe und 1 bis 6 Oxyethylgruppen,
- lineare Alkansulfonate mit 8 bis 24 C-Atomen,
- lineare Alpha-Olefinsulfonate mit 8 bis 24 C-Atomen,
- Alpha-Sulfofettsäuremethylester von Fettsäuren mit 8 bis 30 C-Atomen,
- Alkylsulfate und Alkylpolyglykolethersulfate der Formel $R-O(CH_2-CH_2O)_x-OSO_3H$, in der R eine bevorzugt lineare Alkylgruppe mit 8 bis 30 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 8 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen,
- Alkyl- und/oder Alkenyletherphosphate der Formel (III),

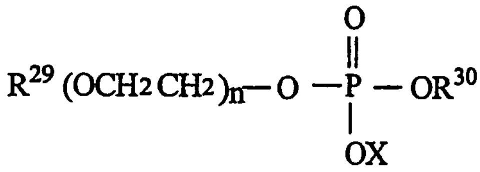

(III)

- in der $R^{29}$ bevorzugt für einen aliphatischen Kohlenwasserstoffrest mit 8 bis 30 Kohlenstoffatomen, $R^{30}$ für Wasserstoff, einen Rest $(CH_2CH_2O)_nR^{29}$ oder X, n für Zahlen von 1 bis 10 und X für Wasserstoff, ein Alkali- oder Erdalkalimetall oder $NR^{31}R^{32}R^{33}R^{34}$, mit $R^{31}$ bis $R^{34}$ unabhängig voneinander stehend für einen $C_1$ bis $C_4$-Kohlenwasserstoffrest, steht,
- sulfatierte Fettsäurealkylenglykolester der Formel (IV)

$$R^{35}CO(AlkO)_nSO_3M \qquad (IV)$$

in der $R^{35}CO$- für einen linearen oder verzweigten, aliphatischen, gesättigten und/oder ungesättigten Acylrest mit 6 bis 22 C-Atomen, Alk für $CH_2CH_2$, $CHCH_3CH_2$ und/oder $CH_2CHCH_3$, n für Zahlen von 0,5 bis 5 und M für ein Kation steht, wie sie in der DE-OS 197 36 906.5 beschrieben sind,
- Monoglyceridsulfate und Nionoglyceridethersulfate der Formel (V) wie sie beispielsweise in der EP-B 1 0 561 825, der EP-B1 0 561 999, der DE-A1 42 04 700 oder

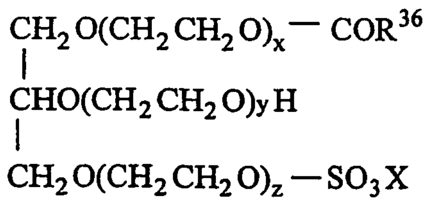

(V)

von A.K.Biswas et al. in J.Am.Oil.Chem.Soc. 37, 171 (1960) und F.U.Ahmed in J.Am.Oil.Chem.Soc.67, 8 (1990) beschrieben worden sind.

Darin steht $R^{36}CO$ für einen linearen oder verzweigten Acylrest mit 6 bis 22 Kohlenstoffatomen, x, y und z in Summe für 0 oder für Zahlen von 1 bis 30, vorzugsweise 2 bis 10, und X für ein Alkali- oder Erdalkalimetall steht. Typische Beispiele für im Sinne der Erfindung geeignete Monoglycerid(ether)sulfate sind die Umsetzungsprodukte von Laurinsäuremonoglycerid, Kokosfettsäuremonoglycerid, Palmitinsäuremonoglycerid, Stearinsäuremonoglycerid, Ölsäuremonoglycerid und Talgfettsäuremonöglycerid sowie deren Ethylenoxidaddukte mit Schwefeltrioxid oder Chlorsulfonsäure in Form ihrer Natriumsalze. Vorzugsweise werden Monoglyceridsulfate der Formel (V) eingesetzt, in der $R^{36}CO$ für einen linearen Acylrest mit 8 bis 18 Kohlenstoffatomen steht

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül und Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen.

Als zwitterionische Tenside werden solche oberflächenaktiven Verbindungen bezeichnet, die im Molekül mindestens eine quartäre Ammoniumgruppe und mindestens eine $-COO^{(-)}$- oder $-SO_3^{(-)}$ -Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Unter ampholytischen Tensiden werden solche oberflächenaktiven Verbindungen verstanden, die außer einer $C_8$ - $C_{24}$ - Alkyl- oder -Acylgruppe im Molekül mindestens eine freie Aminogruppe und mindestens eine -COOH- oder $-SO_3H$-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe. Besonders bevorzugte ampholytische Tenside sind das N-Kokosalkylaminopropionat, das Kokosacylaminoethylaminopropionat und das $C_{12}$ - $C_{18}$ - Acylsarcosin.

Nichtionische Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise

- Anlagerungsprodukte von 2 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe,
- mit einem Methyl- oder $C_2$ - $C_6$ - Alkylrest endgruppenverschlossene Anlagerungsprodukte von 2 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe, wie beispielsweise die unter den Verkaufsbezeichnungen Dehydol® LS, Dehydol® LT (Cognis) erhältlichen Typen,
- $C_{12}$-$C_{30}$-Fettsäuremono- und -diester von Anlagerungsprodukten von 1 bis 30 Mol Ethylenoxid an Glycerin,
- Anlagerungsprodukte von 5 bis 60 Mol Ethylenoxid an Rizinusöl und gehärtetes Rizinusöl,
- Polyolfettsäureester, wie beispielsweise das Handelsprodukt Hydagen® HSP (Cognis) oder Sovermol - Typen (Cognis),
- alkoxilierte Triglyceride,
- alkoxilierte Fettsäurealkylester der Formel (VI)

$$R^{37}CO\text{-}(OCH_2CHR^{38})_wOR^{39} \qquad (VI),$$

  in der $R^{37}CO$ für einen linearen oder verzweigten, gesättigten und/oder ungesättigten Acylrest mit 6 bis 22 Kohlenstoffatomen, $R^{38}$ für Wasserstoff oder Methyl, $R^{39}$ für lineare oder verzweigte Alkylreste mit 1 bis 4 Kohlenstoffatomen und w für Zahlen von 1 bis 20 steht,
- Aminoxide,
- Hydroxymischether, wie sie beipielsweise in der DE-OS 19738866 beschrieben sind,
- Sorbitanfettsäureester und Anlagerungeprodukte von Ethylenoxid an Sorbitanfettsäureester wie beispielsweise die Polysorbate,
- Zuckerfettsäureester und Anlagerungsprodukte von Ethylenoxid an Zuckerfettsäureester,
- Anlagerungsprodukte von Ethylenoxid an Fettsäurealkanolamide und Fettamine,
- Fettsäure-N-alkylglucamide,
- Alkylpolygykoside entsprechend der allgemeinen Formel $RO\text{-}(Z)_x$ wobei R für Alkyl, Z für Zucker sowie x für die Anzahl der Zuckereinheiten steht. Die erfindungsgemäß verwendbaren Alkylpolyglykoside können lediglich einen bestimmten Alkylrest R enthalten. Üblicherweise werden diese Verbindungen aber ausgehend von natürlichen Fetten und Ölen oder Mineralölen hergestellt. In diesem Fall liegen als Alkylreste R Mischungen entsprechend den Ausgangsverbindungen bzw. entsprechend der jeweiligen Aufarbeitung dieser Verbindungen vor.

Besonders bevorzugt sind solche Alkylpolyglykoside, bei denen R

- im wesentlichen aus $C_8$- und $C_{10}$-Alkylgruppen,
- im wesentlichen aus $C_{12}$- und $C_{14}$-Alkylgruppen,
- im wesentlichen aus $C_8$- bis $C_{16}$-Alkylgruppen oder
- im wesentlichen aus $C_{12}$- bis $C_{16}$-Alkylgruppen oder
- im wesentlichen aus $C_{16}$ bis $C_{18}$-Alkylgruppen besteht.

Als Zuckerbaustein Z können beliebige Mono- oder Oligosaccharide eingesetzt werden. Üblicherweise werden Zucker mit 5 bzw. 6 Kohlenstoffatomen sowie die entsprechenden Oligosaccharide eingesetzt. Solche Zucker sind beispielsweise Glucose, Fructose, Galactose, Arabinose, Ribose, Xylose, Lyxose, Allose, Altrose, Mannose, Gulose, Idose, Talose und Sucrose. Bevorzugte Zuckerbausteine sind Glucose, Fructose, Galactose, Arabinose und Sucrose; Glucose ist besonders bevorzugt.

Die erfindungsgemäß verwendbaren Alkylpolyglykoside enthalten im Schnitt 1,1 bis 5 Zuckereinheiten. Alkylpolyglykoside mit x-Werten von 1,1 bis 2,0 sind bevorzugt. Ganz besonders bevorzugt sind Alkylglykoside, bei denen x 1,1 bis 1,8 beträgt.

Auch die alkoxylierten Homologen der genannten Alkylpolyglykoside können erfmdungsgemäß eingesetzt werden. Diese Homologen können durchschnittlich bis zu 10 Ethylenoxid- und/oder Propylenoxideinheiten pro Alkylglykosideinheit enthalten.

Als bevorzugte nichtionische Tenside haben sich die Alkylenoxid-Anlagerungsprodukte an gesättigte lineare Fettalkohole und Fettsäuren mit jeweils 2 bis 30 Mol Ethylenoxid pro Mol Fettalkohol bzw. Fettsäure erwiesen. Zubereitungen mit hervorragenden Eigenschaften werden ebenfalls erhalten, wenn sie als nichtionische Tenside Fettsäureester von ethoxyliertem Glycerin enthalten.

Diese Verbindungen sind durch die folgenden Parameter gekennzeichnet. Der Alkylrest R enthält 6 bis 22 Kohlenstoffatome und kann sowohl linear als auch verzweigt sein. Bevorzugt sind primäre lineare und in 2-Stellung methylverzweigte aliphatische Reste. Solche Alkylreste sind beispielsweise 1-Octyl, 1-Decyl, 1-Lauryl, 1-Myristyl, 1-Cetyl und 1-Stearyl. Besonders bevorzugt sind 1-Octyl, 1-Decyl, 1-Lauryl, 1-Myristyl. Bei Verwendung sogenannter "Oxo-Alkohole" als Ausgangsstoffe überwiegen Verbindungen mit einer ungeraden Anzahl von Kohlenstoffatomen in der Alkylkette.

Bei den als Tensid eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so dass man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Die Tenside werden in Mengen von 0,1 - 45 Gew.%, bevorzugt 1 - 30 Gew.% und ganz besonders bevorzugt von 1 - 15 Gew.%, bezogen auf das gesamte Mittel, eingesetzt.

In einer bevorzugten Ausführungsform können nicht-ionische, zwitterionische und/oder amphotere Tenside sowie deren Mischungen bevorzugt sein.

Erfindungsgemäß bevorzugt sind ebenfalls kationische Tenside vom Typ der quartären Ammoniumverbindungen, der Esterquats und der Amidoamine. Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z.B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27 und Quaternium-83 bekannten Imidazolium-Verbindungen. Die langen Alkylketten der oben genannten Tenside weisen bevorzugt 10 bis 18 Kohlenstoffatome auf

Bei Esterquats handelt es sich um bekannte Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierten Estersalzen von Fettsäuren mit 1,2-Dihydroxypropyldialkylaminen. Solche Produkte werden beispielsweise unter den Warenzeichen Stepantex® , Dehyquart® und Armocare® vertrieben. Die Produkte Armocare® VGH-70, ein N,N-Bis(2-Palmitoyloxyethyl)dimethylammoniumchlorid, sowie Dehyquart® F-75, Dehyquart® C-4046, Dehyquart® L80 und Dehyquart® AU-35 sind Beispiele für solche Esterquats.

Die Alkylamidoamine werden üblicherweise durch Amidierung natürlicher oder synthetischer Fettsäuren und Fettsäureschnitte mit Dialkylaminoaminen hergestellt. Eine erfindungsgemäß besonders geeignete Verbindung aus dieser Substanzgruppe stellt das unter der Bezeichnung Tegoamid® S 18 im Handel erhältliche Stearamidopropyldimethylamin dar.

Die kationischen Tenside sind in den erfindungsgemäß verwendeten Mitteln bevorzugt in Mengen von 0,05 bis 10 Gew.-%, bezogen auf das gesamte Mittel, enthalten. Mengen von 0,1 bis 5 Gew.-% sind besonders bevorzugt.

In einer weiteren bevorzugten Ausführungsform kann die Wirkung des erfindungsgemäßen Wirkstoffes durch Emulgatoren gesteigert werden. Solche Emulgatoren sind beispielsweise

- Anlagerungsprodukte von 4 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- $C_{12}$-$C_{22}$-Fettsäuremono- und -diester von Anlagerungsprodukten von 1 bis 30 Mol Ethylenoxid an Polyole mit 3 bis 6 Kohlenstoffatomen, insbesondere an Glycerin,
- Ethylenoxid- und Polyglycerin-Anlagerungsprodukte an Methylglucosid-Fettsäureester, Fettsäurealkanolamide und

Fettsäureglucamide,

- $C_8$-$C_{22}$-Alkylmono- und -oligoglycoside und deren ethoxylierte Analoga, wobei Oligomerisierungsgrade von 1,1 bis 5, insbesondere 1,2 bis 2,0, und Glucose als Zuckerkomponente bevorzugt sind,
- Gemische aus Alkyl-(oligo)-glucosiden und Fettalkoholen zum Beispiel das im Handel erhältliche Produkt Montanov® 68,
- Anlagerungsprodukte von 5 bis 60 Mol Ethylenoxid an Rizinusöl und gehärtetes Rizinusöl,
- Partialester von Polyolen mit 3-6 Kohlenstoffatomen mit gesättigten Fettsäuren mit 8 bis 22 C-Atomen,
- Sterine. Als Sterine wird eine Gruppe von Steroiden verstanden, die am C-Atom 3 des Steroid-Gerüstes eine Hydroxylgruppe tragen und sowohl aus tierischem Gewebe (Zoosterine) wie auch aus pflanzlichen Fetten (Phytosterine) isoliert werden. Beispiele für Zoosterine sind das Cholesterin und das Lanosterin. Beispiele geeigneter Phytosterine sind Ergosterin, Stigmasterin und Sitosterin. Auch aus Pilzen und Hefen werden Sterine, die sogenannten Mykosterine, isoliert.
- Phospholipide. Hierunter werden vor allem die Glucose-Phospolipide, die z.B. als Lecithine bzw. Phospahtidylcholine aus z.B. Eidotter oder Pflanzensamen (z.B. Sojabohnen) gewonnen werden, verstanden.
- Fettsäureester von Zuckern und Zuckeralkoholen, wie Sorbit
- Polyglycerine und Polyglycerinderivate wie beispielsweise Polyglycerinpoly-12-hydroxystearat (Handelsprodukt Dehymuls® PGPH)
- Lineare und verzweigte Fettsäuren mit 8 bis 30 C - Atomen und deren Na-, K-, Ammonium-, Ca-, Mg- und Zn - Salze.

Die erfindungsgemäßen Mittel enthalten die Emulgatoren bevorzugt in Mengen von 0,1 - 25 Gew.-%, insbesondere 0,5-15 Gew.-%, bezogen auf das gesamte Mittel.

Bevorzugt können die erfindungsgemäßen Zusammensetzungen mindestens einen nichtionogenen Emulgator mit einem HLB-Wert von 8 bis 18, gemäß den im Römpp-Lexikon Chemie (Hrg. J. Falbe, M.Regitz), 10. Auflage, Georg Thieme Verlag Stuttgart, New York, (1997), Seite 1764, aufgeführten Definitionen enthalten. Nichtionogene Emulgatoren mit einem HLB-Wert von 10-15 können erfindungsgemäß besonders bevorzugt sein.

In weiteren bevorzugten Ausführungsform der erfindungsgemäßen Lehre kann die Wirkung mit Polymeren weiter gesteigert werden. Unter Polymeren sind sowohl natürliche als auch synthetische Polymere, welche anionisch, kationisch, amphoter geladen oder nichtionisch sein können, zu verstehen.

Unter kationischen Polymeren sind Polymere zu verstehen, welche in der Haupt- und/oder Seitenkette Gruppen aufweisen, welche "temporär" oder "permanent" kationisch sein kann. Als "permanent kationisch" werden erfindungsgemäß solche Polymere bezeichnet, die unabhängig vom pH-Wert des Mittels eine kationische Gruppe aufweisen. Dies sind in der Regel Polymere, die ein quartäres Stickstoffatom, beispielsweise in Form einer Ammoniumgruppe, enthalten. Bevorzugte kationische Gruppen sind quartäre Ammoniumgruppen. Insbesondere solche Polymere, bei denen die quartäre Ammoniumgruppe über eine $C_{1-4}$-Kohlenwasserstoffgruppe an eine aus Acrylsäure, Methacrylsäure oder deren Derivaten aufgebaute Polymerhauptkette gebunden sind, haben sich als besonders geeignet erwiesen.

Homopolymere der allgemeinen Formel (VII),

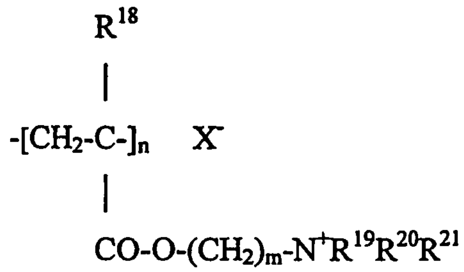

(VII)

in der $R^{18}$ = -H oder -$CH_3$ ist, $R^{19}$, $R^{20}$ und $R^{21}$ unabhängig voneinander ausgewählt sind aus $C_{1-4}$-Alkyl-, -Alkenyl- oder Hydroxyalkylgruppen, m =1, 2, 3 oder 4, n eine natürliche Zahl und $X^-$ ein physiologisch verträgliches organisches oder anorganisches Anion ist, sowie Copolymere, bestehend im wesentlichen aus den in Formel (VII) aufgeführten Monomereinheiten sowie nichtionogenen Monomereinheiten, sind besonders bevorzugte kationische Polymere. Im Rahmen dieser Polymere sind diejenigen erfindungsgemäß bevorzugt, für die mindestens eine der folgenden Bedingungen gilt:

- $R^{18}$ steht für eine Methylgruppe
- $R^{19}$, $R^{20}$ und $R^{21}$ stehen für Methylgruppen
- m hat den Wert 2.

  Als physiologisch verträgliches Gegenionen $X^-$ kommen beispielsweise Halogenidionen, Sulfationen, Phosphationen, Methosulfationen sowie organische Ionen wie Lactat-, Citrat-, Tartrat- und Acetationen in Betracht. Bevorzugt sind Halogenidionen, insbesondere Chlorid.
Ein besonders geeignetes Homopolymer ist das, gewünschtenfalls vemetzte, Poly(methacryloyloxyethyltrimethylammoniumchlorid) mit der INCI-Bezeichnung Polyquaternium-37. Die Vernetzung kann gewünschtenfalls mit Hilfe mehrfach olefinisch ungesättigter Verbindungen, beispielsweise Divinylbenzol, Tetraallyloxyethan, Methylenbisacrylamid, Diallylether, Polyallylpolyglycerylether, oder Allylethern von Zuckern oder Zuckerderivaten wie Erythritol, Pentaerythritol, Arabitol, Mannitol, Sorbitol, Sucrose oder Glucose erfolgen. Methylenbisacrylamid ist ein bevorzugtes Vernetzungsagens.
Das Homopolymer wird bevorzugt in Form einer nichtwässrigen Polymerdispersion, die einen Polymeranteil nicht unter 30 Gew.-% aufweisen sollte, eingesetzt. Solche Polymedispersionen sind unter den Bezeichnungen Salcare® SC 95 (ca. 50 % Polymeranteil, weitere Komponenten: Mineralöl (INCI-Bezeichnung: Mineral Oil) und Tridecylpolyoxypropylen-polyoxyethylen-ether (INCI-Bezeichnung: PPG-1-Trideceth-6)) und Salcare® SC 96 (ca. 50 % Polymeranteil, weitere Komponenten: Mischung von Diestern des Propylenglykols mit einer Mischung aus Capryl- und Caprinsäure (INCI-Bezeichnung: Propylene Glycol Dicaprylate/Dicaprate) und Tridecyl-polyoxypropylen-polyoxyethylenether (INCI-Bezeichnung: PPG-1-Trideceth-6)) im Handel erhältlich.

Copolymere mit Monomereinheiten gemäß Formel (VI) enthalten als nichtionogene Monomereinheiten bevorzugt Acrylamid, Methacrylamid, Acrylsäure-$C_{1-4}$-alkylester und Methacrylsäure-$C_{1-4}$-alkylester. Unter diesen nichtionogenen Monomeren ist das Acrylamid besonders bevorzugt. Auch diese Copolymere können, wie im Falle der Homopolymere oben beschrieben, vemetzt sein. Ein erfindungsgemäß bevorzugtes Copolymer ist das vemetzte Acrylamid-Methacryloyloxyethyltrimethylammoniumchlorid-Copolymer. Solche Copolymere, bei denen die Monomere in einem Gewichtsverhältnis von etwa 20:80 vorliegen, sind im Handel als ca. 50 %ige nichtwässrige Polymerdispersion unter der Bezeichnung Salcare® SC 92 erhältlich.
Weitere bevorzugte kationische Polymere sind beispielsweise

- quaternisierte Cellulose-Derivate, wie sie unter den Bezeichnungen Celquat® und Polymer JR® im Handel erhältlich sind. Die Verbindungen Celquat® H 100, Celquat® L 200 und Polymer JR® 400 sind bevorzugte quaternierte Cellulose-Derivate,
- kationische Alkylpolyglycoside gemäß der DE-PS 44 13 686,
- kationiserter Honig, beispielsweise das Handelsprodukt Honeyquat® 50,
- kationische Guar-Derivate, wie insbesondere die unter den Handelsnamen Cosmedia® Guar und Jaguar® vertriebenen Produkte,
- Polysiloxane mit quaternären Gruppen, wie beispielsweise die im Handel erhältlichen Produkte Q2-7224 (Hersteller: Dow Corning; ein stabilisiertes Trimethylsilylamodimethicon), Dow Corning® 929 Emulsion (enthaltend ein hydroxylaminomodifiziertes Silicon, das auch als Amodimethicone bezeichnet wird), SM-2059 (Hersteller: General Electric), SLM-55067 (Hersteller: Wacker) sowie Abil® -Quat 3270 und 3272 (Hersteller: Th. Goldschmidt; diquatemäre Polydimethylsiloxane, Quatemium-80),
- polymere Dimethyldiallylammoniumsalze und deren Copolymere mit Estern und Amiden von Acrylsäure und Methacrylsäure. Die unter den Bezeichnungen Merquat® 100 (Poly(dimethyldiallylammoniumchlorid)) und Merquat® 550 (Dimethyldiallylammoniumchlorid-Acrylamid-Copolymer) im Handel erhältlichen Produkte sind Beispiele für solche kationischen Polymere,
- Copolymere des Vinylpyrrolidons mit quaternierten Derivaten des Dialkylaminoalkylacrylats und -methacrylats, wie beispielsweise mit Diethylsulfat quaternierte Vinylpyrrolidon-Dimethylaminoethylmethacrylat-Copolymere. Solche Verbindungen sind unter den Bezeichnungen Gafquat® 734 und Gafquat® 755 im Handel erhältlich,
- Vinylpyrrolidon-Vinylimidazoliummethochlorid-Copolymere, wie sie unter den Bezeichnungen Luviquat® FC 370, FC 550, FC 905 und HM 552 angeboten werden.
- quaternierter Polyvinylalkohol,

sowie die unter den Bezeichnungen

- Polyquaternium 2,
- Polyquaternium 17,
- Polyquaternium 18 und
- Polyquaternium 27 bekannten Polymeren mit quartären Stickstoffatomen in der Polymerhauptkette.

Gleichfalls als kationische Polymere eingesetzt werden können die unter den Bezeichnungen Polyquatemium-24 (Handelsprodukt z. B. Quatrisoft® LM 200), bekannten Polymere. Ebenfalls erfindungsgemäß verwendbar sind die Copolymere des Vinylpyrrolidons, wie sie als Handelsprodukte Copolymer 845 (Hersteller: ISP), Gaffix® VC 713 (Hersteller: ISP), Gafquat® ASCP 1011, Gafquat® HS 110, Luviquat® 8155 und Luviquat® MS 370 erhältlich sind.

Weitere erfindungsgemäße kationische Polymere sind die sogenannten "temporär kationischen" Polymere. Diese Polymere enthalten üblicherweise eine Aminogruppe, die bei bestimmten pH-Werten als quartäre Ammoniumgruppe und somit kationisch vorliegt. Bevorzugt sind beispielsweise Chitosan und dessen Derivate, wie sie beispielsweise unter den Handelsbezeichnungen Hydagen® CMF, Hydagen® HCMF, Kytamer® PC und Chitolam® NB/101 im Handel frei verfügbar sind. Chitosane sind deacetylierte Chitine, die in unterschiedlichen Deacetylierungsgraden und unterschiedlichen Abbaugraden (Molekulargewichten) im Handel erhältlich sind. Ihre Herstellung ist z.B. in DE 44 40 625 A1 und in DE 1 95 03 465 A1 beschrieben.

Besonders gut geeignete Chitosane weisen einen Deacetylierungsgrad von wenigstens 80 % und ein Molekulargewicht von $5 \cdot 10^5$ bis $5 \cdot 10^6$ (g/mol) auf.

Zur Herstellung erfindungsgemäßer Zubereitungen muss das Chitosan in die Salzform überführt werden. Dies kann durch Auflösen in verdünnten wässrigen Säuren erfolgen. Als Säuren sind sowohl Mineralsäuren wie z.B. Salzsäure, Schwefelsäure und Phosphorsäure als auch organische Säuren, z.B. niedermolekulare Carbonsäuren, Polycarbonsäuren und Hydroxycarbonsäuren geeignet. Weiterhin können auch höhermolekulare Alkylsulfonsäuren oder Alkylschwefelsäuren oder Organophosphorsäuren verwendet werden, soweit diese die erforderliche physiologische Verträglichkeit aufweisen. Geeignete Säuren zur Überführung des Chitosans in die Salzform sind z.B. Essigsäure, Glycolsäure, Weinsäure, Apfelsäure, Citronensäure, Milchsäure, 2-Pyrrolidinon-5-carbonsäure, Benzoesäure oder Salicylsäure. Bevorzugt werden niedermolekulare Hydroxycarbonsäuren wie z.B. Glycolsäure oder Milchsäure verwendet.

Bei den anionischen Polymeren, welche die Wirkung des erfindungsgemäßen Wirkstoffes unterstützen können, handelt es sich um ein anionische Polymere, welche Carboxylat- und/oder Sulfonatgruppen aufweisen. Beispiele für anionische Monomere, aus denen derartige Polymere bestehen können, sind Acrylsäure, Methacrylsäure, Crotonsäure, Maleinsäureanhydrid und 2-Acrylamido-2-methylpropansulfonsäure. Dabei können die sauren Gruppen ganz oder teilweise als Natrium-, Kalium-, Ammonium-, Mono- oder Triethanolammonium-Salz vorliegen. Bevorzugte Monomere sind 2-Acrylamido-2-methylpropansulfonsäure und Acrylsäure.

Als ganz besonders wirkungsvoll haben sich anionische Polymere erwiesen, die als alleiniges oder Co-Monomer 2-Acrylamido-2-methylpropansulfonsäure enthalten, wobei die Sulfonsäuregruppe ganz oder teilweise als Natrium-, Kalium-, Ammonium-, Mono- oder Triethanolammonium-Salz vorliegen kann.

Besonders bevorzugt ist das Homopolymer der 2-Acrylamido-2-methylpropansulfonsäure, das beispielsweise unter der Bezeichnung Rheothik® 11-80 im Handel erhältlich ist.

Innerhalb dieser Ausführungsform kann es bevorzugt sein, Copolymere aus mindestens einem anionischen Monomer und mindestens einem nichtionogenen Monomer einzusetzen. Bezüglich der anionischen Monomere wird auf die oben aufgeführten Substanzen verwiesen. Bevorzugte nichtionogene Monomere sind Acrylamid, Methacrylamid, Acrylsäureester, Methacrylsäureester, Vinylpyrrolidon, Vinylether und Vinylester.

Bevorzugte anionische Copolymere sind Acrylsäure-Acrylamid-Copolymere sowie insbesondere Polyacrylamidcopolymere mit Sulfonsäuregruppen-haltigen Monomeren. Ein besonders bevorzugtes anionisches Copolymer besteht aus 70 bis 55 Mol-% Acrylamid und 30 bis 45 Mol-% 2-Acrylamido-2-methylpropansulfonsäure, wobei die Sulfonsäuregruppe ganz oder teilweise als Natrium-, Kalium-, Ammonium-, Mono- oder Triethanolammonium-Salz vorliegt. Dieses Copolymer kann auch vernetzt vorliegen, wobei als Vemetzungsagentien bevorzugt polyolefinisch ungesättigte Verbindungen wie Tetraallyloxyethan, Allylsucrose, Allylpentaerythrit und Methylen-bisacrylamid zum Einsatz kommen. Ein solches Polymer ist in dem Handelsprodukt Sepigel® 305 der Firma SEPPIC enthalten. Die Verwendung dieses Compounds, das neben der Polymerkomponente eine Kohlenwasserstoffmischung ($C_{13}$-$C_{14}$-Isoparaffin) und einen nichtionogenen Emulgator (Laureth-7) enthält, hat sich im Rahmen der erfindungsgemäßen Lehre als besonders vorteilhaft erwiesen.

Auch die unter der Bezeichnung Simulgel® 600 als Compound mit Isohexadecan und Polysorbat-80 vertriebenen Natriumacryloyldimethyltaurat-Copolymere haben sich als erfindungsgemäß besonders wirksam erwiesen.

Ebenfalls bevorzugte anionische Homopolymere sind unvemetzte und vernetzte Polyacrylsäuren. Dabei können Allylether von Pentaerythrit, von Sucrose und von Propylen bevorzugte Vernetzungsagentien sein. Solche Verbindungen sind beispielsweise unter dem Warenzeichen Carbopol® im Handel erhältlich.

Copolymere aus Maleinsäureanhydrid und Methylvinylether, insbesondere solche mit Vernetzungen, sind ebenfalls farberhaltende Polymere. Ein mit 1,9-Decadiene vernetztes Maleinsäure-Methylvinylether-Copolymer ist unter der Bezeichnungg Stabileze® QM im Handel erhältlich.

Weiterhin können als Polymere zur Steigerung der Wirkung des erfindungsgemäßen Wirkstoffes amphotere Polymere als Bestandteil eingesetzt. Unter dem Begriff amphotere Polymere werden sowohl solche Polymere, die im Molekül sowohl freie Aminogruppen als auch freie -COOH- oder $SO_3H$-Gruppen enthalten und zur Ausbildung innerer Salze befähigt sind, als auch zwitterionische Polymere, die im Molekül quartäre Ammoniumgruppen und $-COO^-$- oder $-SO_3^-$-Gruppen enthalten, und solche Polymere zusammengefasst, die -COOH- oder $SO_3H$-Gruppen und quartäre Ammoniumgruppen enthalten.

Ein Beispiel für ein erfindungsgemäß einsetzbares Amphopolymer ist das unter der Bezeichnung Amphomer®

erhältliche Acrylharz, das ein Copolymeres aus tert.-Butylaminoethylmethacrylat, N-(1,1,3,3-Tetramethylbutyl)acrylamid sowie zwei oder mehr Monomeren aus der Gruppe Acrylsäure, Methacrylsäure und deren einfachen Estern darstellt.

Weitere erfindungsgemäß einsetzbare amphotere Polymere sind die in der britischen Offenlegungsschrift 2 104 091, der europäischen Offenlegungsschrift 47 714, der europäischen Offenlegungsschrift 217 274, der europäischen Offenlegungsschrift 283 817 und der deutschen Offenlegungsschrift 28 17 369 genannten Verbindungen.

Bevorzugt eingesetzte amphotere Polymere sind solche Polymerisate, die sich im wesentlichen zusammensetzen aus

(a) Monomeren mit quartären Ammoniumgruppen der allgemeinen Formel (VIII),

$$R^{22}\text{-}CH\text{=}CR^{23}\text{-}CO\text{-}Z\text{-}(C_nH_{2n})\text{-}N^{(+)}R^{24}R^{25}R^{26}\ A^{(-)} \qquad \text{(VIII)}$$

in der $R^{22}$ und $R^{23}$ unabhängig voneinander stehen für Wasserstoff oder eine Methylgruppe und $R^{24}$, $R^{25}$ und $R^{26}$ unabhängig voneinander für Alkylgruppen mit 1 bis 4 Kohlenstoff-Atomen, Z eine NH-Gruppe oder ein Sauerstoffatom, n eine ganze Zahl von 2 bis 5 und $A^{(-)}$ das Anion einer organischen oder anorganischen Säure ist und

(b) monomeren Carbonsäuren der allgemeinen Formel (IX),

$$R^{27}\text{-}CH\text{=}CR^{28}\text{-}COOH \qquad \text{(IX)}$$

in denen $R^{27}$ und $R^{28}$ unabhängig voneinander Wasserstoff oder Methylgruppen sind.

Diese Verbindungen können sowohl direkt als auch in Salzform, die durch Neutralisation der Polymerisate, beispielsweise mit einem Alkalihydroxid, erhalten wird, erfindungsgemäß eingesetzt werden. Bezüglich der Einzelheiten der Herstellung dieser Polymerisate wird ausdrücklich auf den Inhalt der deutschen Offenlegungsschrift 39 29 973 Bezug genommen. Ganz besonders bevorzugt sind solche Polymerisate, bei denen Monomere des Typs (a) eingesetzt werden, bei denen $R^{24}$, $R^{25}$ und $R^{26}$ Methylgruppen sind, Z eine NH-Gruppe und $A^{(-)}$ ein Halogenid-, Methoxysulfat- oder Ethoxysulfat-Ion ist; Acrylamidopropyl-trimethyl-ammoniumchlorid ist ein besonders bevorzugtes Monomeres (a). Als Monomeres (b) für die genannten Polymerisate wird bevorzugt Acrylsäure verwendet. Die erfindungsgemäßen Mittel können weiterhin nichtionogene Polymere enthalten.

Geeignete nichtionogene Polymere sind beispielsweise:

- Vinylpyrrolidon/Vinylester-Copolymere, wie sie beispielsweise unter dem Warenzeichen Luviskol® (BASF) vertrieben werden. Luviskol® VA 64 und Luviskol® VA 73, jeweils Vinylpyrrolidon/Vinylacetat-Copolymere, sind ebenfalls bevorzugte nichtionische Polymere.
- Celluloseether, wie Hydroxypropylcellulose, Hydroxyethylcellulose und Methylhydroxypropylcellulose, wie sie beispielsweise unter den Warenzeichen Culminal® und Benecel® (AQUALON) vertrieben werden.
- Schellack
- Polyvinylpyrrolidone, wie sie beispielsweise unter der Bezeichnung Luviskol® (BASF) vertrieben werden.
- Siloxane. Diese Siloxane können sowohl wasserlöslich als auch wasserunlöslich sein. Geeignet sind sowohl flüchtige als auch nichtflüchtige Siloxane, wobei als nichtflüchtige Siloxane solche Verbindungen verstanden werden, deren Siedepunkt bei Normaldruck oberhalb von 200 °C liegt. Bevorzugte Siloxane sind Polydialkylsiloxane, wie beispielsweise Polydimethylsiloxan, Polyalkylarylsiloxane, wie beispielsweise Polyphenylmethylsiloxan, ethoxylierte Polydialkylsiloxane sowie Polydialkylsiloxane, die Amin- und/oder Hydroxy-Gruppen enthalten.
- Glycosidisch substituierte Silicone gemäß der EP 0612759 B1.

Es ist erfindungsgemäß auch möglich, dass die verwendeten Zubereitungen mehrere, insbesondere zwei verschiedene Polymere gleicher Ladung und/oder jeweils ein ionisches und ein amphoteres und/oder nicht ionisches Polymer enthalten.

Unter dem Begriff Polymer sind erfindungsgemäß ebenfalls spezielle Zubereitungen von Polymeren wie sphärische Polymerpulver zu verstehen. Es sind verschiedene Verfahren bekannt, solche Mikrokugeln aus verschiedenen Monomeren herzustellen, z.B. durch spezielle Polymerisationsverfahren oder durch Auflösen des Polymeren in einem Lösungsmittel und Versprühen in ein Medium, in dem das Lösungsmittel verdunsten oder aus den Teilchen herausdiffundieren kann. Ein solches Verfahren ist z.B. aus EP 466 986 B1 bekannt. Geeignete Polymerisate sind z.B. Polycarbonate, Polyurethane, Polyacrylate, Polyolefine, Polyester oder Polyamide. Besonders geeignet sind solche sphärischen Polymerpulver, deren Primärpartikeldurchmesser unter 1 $\mu$m liegt. Solche Produkte auf Basis eines Polymethacrylat-Copolymers sind z.B. unter dem Warenzeichen Polytrap® QS-6603 (Dow Coming) im Handel. Andere Polymerpulver, z.B. auf Basis von Polyamiden (Nylon 6, Nylon 12) sind mit einer Teilchengröße von 2 - 10 $\mu$m (90 %) und einer

spezifischen Oberfläche von ca. 10 $m^2$/g unter der Handelsbezeichnung Orgasol® 2002 DU Nat Cos (Atochem S.A., Paris) erhältlich. Weitere sphärische Polymerpulver, die für den erfindungsgemäßen Zweck geeignet sind, sind z.B. die Polymethacrylate (Micropearl M) von SEPPIC oder (Plastic Powder A) von NIKKOL, die Styrol-Divinylbenzol-Copolymeren (Plastic Powder FP) von NIKKOL, die Polyethylen- und Polypropylen-Pulver (ACCUREL EP 400) von AKZO, oder auch Silikonpolymere (Silicone Powder X2-1605) von Dow Corning oder auch sphärische Cellulosepulver.

Die Polymere sind in den erfindungsgemäß verwendeten Mitteln bevorzugt in Mengen von 0,01 bis 10 Gew.-%, bezogen auf das gesamte Mittel, enthalten. Mengen von 0,1 bis 5, insbesondere von 0,1 bis 3 Gew.-%, sind besonders bevorzugt.

In einer weiteren bevorzugten Ausführungsform der Erfindung kann die Wirkung des erfindungsgemäßen Wirkstoffes durch Fettstoffe weiter optimiert werden. Unter Fettstoffen sind zu verstehen Fettsäuren, Fettalkohole, natürliche und synthetische Wachse, welche sowohl in fester Form als auch flüssig in wässriger Dispersion vorliegen können, und natürliche und synthetische kosmetische Ölkomponenten zu verstehen.

Als Fettsäuren können eingesetzt werden lineare und/oder verzweigte, gesättigte und/oder ungesättigte Fettsäuren mit 6-30 Kohlenstoffatomen. Bevorzugt sind Fettsäuren mit 10 - 22 Kohlenstoffatomen. Hierunter wären beispielsweise zu nennen die Isostearinsäuren, wie die Handelsprodukte Emersol® 871 und Emersol® 875, und Isopalmitinsäuren wie das Handelsprodukt Edenor® IP 95, sowie alle weiteren unter den Handelsbezeichnungen Edenor® (Cognis) vertriebenen Fettsäuren. Weitere typische Beispiele für solche Fettsäuren sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z.B. bei der Druckspaltung von natürlichen Fetten und Ölen, bei der Oxidation von Aldehyden aus der Roelen'schen Oxosynthese oder der Dimerisierung von ungesättigten Fettsäuren anfallen. Besonders bevorzugt sind üblicherweise die Fettsäureschnitte, welche aus Cocosöl oder Palmöl erhältlich sind; insbesondere bevorzugt ist in der Regel der Einsatz von Stearinsäure.

Die Einsatzmenge beträgt dabei 0,1 - 15 Gew.%, bezogen auf das gesamte Mittel. In einer bevorzugten Ausfihrungsform beträgt die Menge 0,5 - 10 Gew.%, wobei ganz besonders vorteilhaft Mengen von 1 - 5 Gew.% sind.

Als Fettalkohole können eingesetzt werden gesättigte, ein- oder mehrfach ungesättigte, verzweigte oder unverzweigte Fettalkohole mit $C_6$ - $C_{30}$-, bevorzugt $C_{10}$ - $C_{22}$- und ganz besonders bevorzugt $C_{12}$ - $C_{22}$- Kohlenstoffatomen. Einsetzbar im Sinne der Erfindung sind beispielsweise Decanol, Octanol, Octenol, Dodecenol, Decenol, Octadienol, Dodecadienol, Decadienol, Oleylalkohol, Erucaalkohol, Ricinolalkohol, Stearylalkohol, Isostearylalkohol, Cetylalkohol, Laurylalkohol, Myristylalkohol, Arachidylalkohol, Caprylalkohol, Caprinalkohol, Linoleylalkohol, Linolenylalkohol und Behenylalkohol, sowie deren Guerbetalkohole, wobei diese Aufzählung beispielhaften und nicht limitierenden Charakter haben soll. Die Fettalkohole stammen jedoch von bevorzugt natürlichen Fettsäuren ab, wobei üblicherweise von einer Gewinnung aus den Estern der Fettsäuren durch Reduktion ausgegangen werden kann. Erfindungsgemäß einsetzbar sind ebenfalls solche Fettalkoholschnitte, die durch Reduktion natürlich vorkommender Triglyceride wie Rindertalg, Palmöl, Erdnussöl, Rüböl, Baumwollsaatöl, Sojaöl, Sonnenblumenöl und Leinöl oder aus deren Umesterungsprodukten mit entsprechenden Alkoholen entstehenden Fettsäureestern erzeugt werden, und somit ein Gemisch von unterschiedlichen Fettalkoholen darstellen. Solche Substanzen sind beispielsweise unter den Bezeichnungen Stenol® , z.B. Stenol® 1618 oder Lanette® , z.B. Lanette® O oder Lorol® , z.B. Lorol® C8, Lorol® C14, Lorol® C18, Lorol® C8-18, HD-Ocenol® , Crodacol® , z.B. Crodacol® CS, Novol® , Eutanol® G, Guerbitol® 16, Guerbitol® 18, Guerbitol® 20, Isofol® 12, Isofol® 16, Isofol® 24, Isofol® 36, Isocarb® 12, Isocarb® 16 oder Isocarb® 24 käuflich zu erwerben. Selbstverständlich können erfindungsgemäß auch Wollwachsalkohole, wie sie beispielsweise unter den Bezeichnungen Corona® , White Swan® , Coronet® oder Fluilan® käuflich zu erwerben sind, eingesetzt werden.

Die Fettalkohole werden in Mengen von 0,1 - 20 Gew.-%, bezogen auf die gesamte Zubereitung, bevorzugt in Mengen von 0,1 - 10 Gew.-% eingesetzt.

Als natürliche oder synthetische Wachse können erfindungsgemäß eingesetzt werden feste Paraffine oder Isoparaffine, Carnaubawachse, Bienenwachse, Candelillawachse, Ozokerite, Ceresin, Walrat, Sonnenblumenwachs, Fruchtwachse wie beispielsweise Apfelwachs oder Citruswachs, Microwachse aus PE- oder PP. Derartige Wachse sind beispielsweise erhältlich über die Fa. Kahl & Co., Trittau.

Zu den natürlichen und synthetischen kosmetischen Ölkörpern, welche die Wirkung des erfindungsgemäßen Wirkstoffes steigern können, sind beispielsweise zu zählen:

- pflanzliche Öle. Beispiele für solche Öle sind Sonnenblumenöl, Olivenöl, Sojaöl, Rapsöl, Mandelöl, Jojobaöl, Orangenöl, Weizenkeimöl, Pfirsichkernöl und die flüssigen Anteile des Kokosöls. Geeignet sind aber auch andere Triglyceridöle wie die flüssigen Anteile des Rindertalgs sowie synthetische Triglyceridöle.
- flüssige Paraffinöle, Isoparaffinöle und synthetische Kohlenwasserstoffe sowie Di-n-alkylether mit insgesamt zwischen 12 bis 36 C-Atomen, insbesondere 12 bis 24 C-Atomen, wie beispielsweise Di-n-octylether, Di-n-decylether, Di-n-nonylether, Di-n-undecylether, Di-n-dodecylether, n-Hexyl-n-octylether, n-Octyl-n-decylether, n-Decyln-undecylether, n-Undecyl-n-dodecylether und n-Hexyl-n-Undecylether sowie Di-tert-butylether, Di-iso-pentylether, Di-3-ethyldecylether, tert.-Butyl-n-octylether, isoPentyl-n-octylether und 2-Methyl-pentyl-n-octylether. Die als Handelsprodukte erhältlichen Verbindungen 1,3-Di-(2-ethyl-hexyl)-cyclohexan (Cetiol® S) und Di-n-octylether (Cetiol® OE) können bevorzugt sein.

- Esteröle. Unter Esterölen sind zu verstehen die Ester von $C_6$ - $C_{30}$ - Fettsäuren mit $C_2$ - $C_{30}$ - Fettalkoholen. Bevorzugt sind die Monoester der Fettsäuren mit Alkoholen mit 2 bis 24 C-Atomen. Beispiele für eingesetzte Fettsäurenanteile in den Estern sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z.B. bei der Druckspaltung von natürlichen Fetten und Ölen, bei der Oxidation von Aldehyden aus der Roelen'schen Oxosynthese oder der Dimerisierung von ungesättigten Fettsäuren anfallen. Beispiele für die Fettalkoholanteile in den Esterölen sind Isopropylalkohol, Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaestearylalkohol, Arachylalkohol, Gadoleylalkohol, Behenylalkohol, Erucylalkohol und Brassidylalkohol sowie deren technische Mischungen, die z.B. bei der Hochdruckhydrierung von technischen Methylestern auf Basis von Fetten und Ölen oder Aldehyden aus der Roelen'schen Oxosynthese sowie als Monomerfraktion bei der Dimerisierung von ungesättigten Fettalkoholen anfallen. Erfindungsgemäß besonders bevorzugt sind Isopropylmyristat (Rilanit® IPM), Isononansäure-C16-18-alkylester (Cetiol® SN), 2-Ethylhexylpalmitat (Cegesoft® 24), Stearinsäure-2-ethylhexylester (Cetiol® 868), Cetyloleat, Glycerintricaprylat, Kokosfettalkohol-caprinat/-caprylat (Cetiol® LC), n-Butylstearat, Oleylerucat (Cetiol® J 600), Isopropylpalmitat (Rilanit® IPP), Oleyl Oleate (Cetiol® ), Laurinsäurehexylester (Cetiol® A), Di-n-butyladipat (Cetiol® B), Myristylmyristat (Cetiol® MM), Cetearyl Isononanoate (Cetiol® SN), Ölsäuredecylester (Cetiol® V).
- Dicarbonsäureester wie Di-n-butyladipat, Di-(2-ethylhexyl)-adipat, Di-(2-ethylhexyl)-succinat und Di-isotridecylacelaat sowie Diolester wie Ethylenglykol-dioleat, Ethylenglykol-di-isotridecanoat, Propylenglykol-di(2-ethylhexanoat), Propylenglykol-di-isostearat, Propylenglykol-di-pelargonat, Butandiol-di-isostearat, Neopentylglykoldicaprylat,
- symmetrische, unsymmetrische oder cyclische Ester der Kohlensäure mit Fettalkoholen, beispielsweise beschrieben in der DE-OS 197 56 454, Glycerincarbonat oder Dicaprylylcarbonat (Cetiol® CC),
- Mono,- Di- und Trifettsäureester von gesättigten und/oder ungesättigten linearen und/oder verzweigten Fettsäuren mit Glycerin, wie beispielsweise Monomuls® 90-018, Monomuls® 90-L12 oder Cutina® MD.

Die Einsatzmenge beträgt 0,1 - 50 Gew.% bezogen auf das gesamte Mittel, bevorzugt 0,1 - 20 Gew.% und besonders bevorzugt 0,1 - 15 Gew.% bezogen auf das gesamte Mittel.

Die Gesamtmenge an Öl- und Fettkomponenten in den erfindungsgemäßen Mitteln beträgt üblicherweise 6 - 45 Gew.-%, bezogen auf das gesamte Mittel. Mengen von 10- 35 Gew.-% sind erfindungsgemäß bevorzugt.

Weiterhin hat sich gezeigt, dass die Wirkung des erfindungsgemäßen Wirkstoffes gesteigert werden kann, wenn er mit Hydroxycarbonsäureestern kombiniert wird. Bevorzugte Hydroxycarbonsäureester sind Vollester der Glycolsäure, Milchsäure, Äpfelsäure, Weinsäure oder Citronensäure. Weitere grundsätzlich geeigneten Hydroxycarbonsäureester sind Ester der β-Hydroxypropionsäure, der Tarkronsäure, der D-Gluconsäure, Zuckersäure, Schleimsäure oder Glucuronsäure. Als Alkoholkomponente dieser Ester eignen sich primäre, lineare oder verzweigte aliphatische Alkohole mit 8 - 22 C-Atomen, also z.B. Fettalkohole oder synthetische Fettalkohole. Dabei sind die Ester von $C_{12}$-$C_{15}$-Fettäkoholen besonders bevorzugt. Ester dieses Typs sind im Handel erhältlich, z.B. unter dem Warenzeichen Cosmacol® der EniChem, Augusta Industriale.

Die Einsatzmenge der Hydroxycarbonsäureester beträgt dabei 0,1 - 15 Gew.% bezogen auf das Mittel, bevorzugt 0,1 - 10 Gew.% und ganz besonders bevorzugt 0,1 - 5 Gew.%.

In einer weiteren Ausführungsform der erfindungsgemäßen Mittel kann die Wirkung durch die Verwendung von Proteinhydrolysaten und deren Derivaten weiter gesteigert werden. Proteinhydrolysate sind Produktgemische, die durch sauer, basisch oder enzymatisch katalysierten Abbau von Proteinen (Eiweißen) erhalten werden.

Erfindungsgemäß können Proteinhydrolysate sowohl pflanzlichen als auch tierischen Ursprungs eingesetzt werden.

Tierische Proteinhydrolysate sind beispielsweise Elastin-, Kollagen-, Keratin-, Seiden- und Milcheiweiß-Proteinhydrolysate, die auch in Form von Salzen vorliegen können. Solche Produkte werden beispielsweise unter den Warenzeichen Dehylan® (Cognis), Promois® (Interorgana), Collapuron® (Cognis), Nutrilan® (Cognis), Gelita-Sol® (Deutsche Gelatine Fabriken Stoess & Co), Lexein® (Inolex) und Kerasol® (Croda) vertrieben.

Erfindungsgemäß bevorzugt ist die Verwendung von Proteinhydrolysaten pflanzlichen Ursprungs, z. B. Soja-, Mandel-, Reis-, Erbsen-, Kartoffel- und Weizenproteinhydrolysate. Solche Produkte sind beispielsweise unter den Warenzeichen Gluadin® (Cognis), DiaMin® (Diamalt), Lexein® (Inolex) und Crotein® (Croda) erhältlich.

Wenngleich der Einsatz der Proteinhydrolysate als solche bevorzugt ist, können an deren Stelle gegebenenfalls auch anderweitig erhaltene Aminosäuregemische oder einzelne Aminosäuren wie beispielsweise Arginin, Lysin, Histidin

oder Pyrroglutaminsäure eingesetzt werden. Ebenfalls möglich ist der Einsatz von Derivaten der Proteinhydrolysate, beispielsweise in Form ihrer Fettsäure-Kondensationsprodukte. Solche Produkte werden beispielsweise unter den Bezeichnungen Lamepon® (Cognis), Gluadin® (Cognis), Lexein® (Inolex), Crolastin® (Croda) oder Crotein® (Croda) vertrieben.

Gemäß einer weiteren bevorzugten Ausführungsform enthalten die erfindungsgemäßen Mittel weiterhin mindestens einen Feststoff, insbesondere mindestens einen Fettstoff, in nanopartikulärer Form. Ein solcher Feststoff ist beispielsweise hydriertes Rizinusöl. Die Größe der Nanopartikel liegt dabei bevorzugt bei etwa 100 nm oder darunter.

In einer weiteren Ausführungsform wird der erfindungsgemäß verwendete Wirkstoff gemäß Formel (I) in Mitteln zum Färben keratinischer Fasern eingesetzt. Dabei kann der erfindungsgemäße Wirkstoff prinzipiell dem Färbemittel direkt zugegeben werden oder das Aufbringen des Wirkstoff-haltigen Mittels erfolgt auf die gefärbte keratinische Faser in einem getrennten Schritt entweder direkt im Anschluss an den eigentlichen Färbevorgang oder in getrennten Behandlungen, gegebenenfalls auch Tage oder Wochen nach dem Färbevorgang.

Der Begriff Färbevorgang umfasst dabei alle dem Fachmann bekannten Verfahren, bei denen auf das, gegebenenfalls angefeuchtete, Haar ein Färbemittel aufgebracht wird und dieses entweder für eine Zeit zwischen wenigen Minuten und ca. 45 Minuten auf dem Haar belassen und anschließend mit Wasser oder einem tensidhaltigen Mittel ausgespült wird oder ganz auf dem Haar belassen wird. Es wird in diesem Zusammenhang ausdrücklich auf die bekannten Monographien, z. B. Kh. Schrader, Grundlagen und Rezepturen der Kosmetika, 2. Auflage, Hüthig Buch Verlag, Heidelberg, 1989, verwiesen, die das entsprechende Wissen des Fachmannes wiedergeben.

Wie bereits zuvor erwähnt ist es im Rahmen der erfindungsgemäßen Lehre möglich, den Wirkstoff direkt in die Färbe- oder Tönungsmittel einzuarbeiten.

Die Zusammensetzung des Färbe- oder Tönungsmittels unterliegt keinen prinzipiellen Einschränkungen. Als Farbstoff(vorprodukt)e können

- Oxidationsfarbstoffvorprodukte vom Entwickler- und Kuppler-Typ,
- natürliche und synthetische direktziehende Farbstoffe und
- Vorstufen naturanaloger Farbstoffe, wie Indol- und Indolin-Derivate,

sowie Mischungen von Vertretern einer oder mehrerer dieser Gruppen eingesetzt werden.

Als Oxidationsfarbstoffvorprodukte vom Entwickler-Typ werden üblicherweise primäre aromatische Amine mit einer weiteren, in para- oder ortho-Position befindlichen, freien oder substituierten Hydroxy- oder Aminogruppe, Diaminopyridinderivate, heterocyclische Hydrazone, 4-Aminopyrazolderivate sowie 2,4,5,6-Tetraaminopyrimidin und dessen Derivate eingesetzt. Geeignete Entwicklerkomponenten sind beispielsweise p-Phenylendiamin, p-Toluylendiamin, p-Aminophenol, o-Aminophenol, 1-(2'-Hydroxyethyl)-2,5-diaminobenzol, N,N-Bis-(2-hydroxy-ethyl)-p-phenylendiamia, 2-(2,5-Diaminophenoxy)-ethanol, 4-Amino-3-methylphenol, 2,4,5,6-Tetraaminopyrimidin, 2-Hydroxy-4,5,6-triaminopyrimidin, 4-Hydroxy-2,5,6-triaminopyrimidin, 2,4-Dihydroxy-5,6-diaminopyrimidin, 2-Dimethylamino-4,5,6-triaminopyrimidin, 2-Hydroxymethylamino-4-aminophenol, Bis-(4-aminophenyl)amin, 4-Amino-3-fluorphenol, 2-Aminomethyl-4-aminophenol, 2-Hydroxymethyl-4-aminophenol, 4-Amino-2-((diethylamino)-methyl)-phenol, Bis-(2-hydroxy-5-aminophenyl)-methan, 1,4-Bis-(4-aminophenyl)-diazacycloheptan, 1,3-Bis(N(2-hydroxyethyl)-N(4-aminophenylamino))-2-propanol, 4-Amino-2-(2-hydroxyethoxy)-phenol, 1,10-Bis-(2,5-diaminophenyl)-1,4,7,10-tetraoxadecan sowie 4,5-Diaminopyrazol-Derivate nach EP 0 740 741 bzw. WO 94/08970 wie z. B. 4,5-Diamino-1-(2'-hydroxyethyl)-pyrazol. Besonders vorteilhafte Entwicklerkomponenten sind p-Phenylendiamin, p-Toluylendiamin, p-Aminophenol, 1-(2'-Hydroxyethyl)-2,5-diaminobenzol, 4-Amino-3-methylphenol, 2-Aminomethyl-4-aminophenol, 2,4,5,6-Tetraaminopyrimidin, 2-Hydroxy-4,5,6-triaminopyrimidin, 4-Hydroxy-2,5,6-triaminopyrimidin.

Als Oxidationsfarbstoffvorprodukte vom Kuppler-Typ werden in der Regel m-Phenylendiaminderivate, Naphthole, Resorcin und Resorcinderivate, Pyrazolone und m-Aminophenolderivate verwendet. Beispiele für solche Kupplerkomponenten sind

- m-Aminophenol und dessen Derivate wie beispielsweise 5-Amino-2-methylphenol, 5-(3-Hydroxypropylamino)-2-methylphenol, 3-Amino-2-chlor-6-methylphenol, 2-Hydroxy-4-aminophenoxyethanol, 2,6-Dimethyl-3-aminophenol, 3-Trifluoroacetylamino-2-chlor-6-methylphenol, 5-Amino-4-chlor-2-methylphenol, 5-Amino-4-methoxy-2-methylphenol, 5-(2'-Hydroxyethyl)-amino-2-methylphenol, 3-(Diethylamino)-phenol, N-Cyclopentyl-3-aminophenol, 1,3-Dihydroxy-5-(methylamino)-benzol, 3-(Ethylamino)-4-methylphenol und 2,4-Dichlor-3-aminophenol,
- o-Aminophenol und dessen Derivate,
- m-Diaminobenzol und dessen Derivate wie beispielsweise 2,4-Diaminophenoxyethanol, 1,3-Bis-(2,4-diaminophenoxy)-propan, 1-Methoxy-2-amino-4-(2'-hydroxyethylamino)benzol, 1,3-Bis-(2,4-diaminophenyl)-propan, 2,6-Bis-(2-hydroxyethylamino)-1-methylbenzol und 1-Amino-3-bis-(2'-hydroxyethyl)-aminobenzol,
- o-Diaminobenzol und dessen Derivate wie beispielsweise 3,4-Diaminobenzoesäure und 2,3-Diamino-1-methylbenzol,

- Di- beziehungsweise Trihydroxybenzolderivate wie beispielsweise Resorcin, Resorcinmonomethylether, 2-Methylresorcin, 5-Methylresorcin, 2,5-Dimethylresorcin, 2-Chlorresorcin, 4-Chlorresorcin, Pyrogallol und 1,2,4-Trihydroxybenzol,
- Pyridinderivate wie beispielsweise 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-4-methylpyridin, 2,6-Diaminopyridin, 2,3-Diamino-6-methoxypyridin und 3,5-Diamino-2,6-dimethoxypyridin,
- Naphthalinderivate wie beispielsweise 1-Naphthol, 2-Methyl-1-naphthol, 2-Hydroxymethyl-1-naphthol, 2-Hydroxyethyl-1-naphthol, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin und 2,3-Dihydroxynaphthalin,
- Morpholinderivate wie beispielsweise 6-Hydroxybenzomorpholin und 6-Aminobenzomorpholin,
- Chinoxalinderivate wie beispielsweise 6-Methyl-1,2,3,4-tetrahydrochinoxalin,
- Pyrazolderivate wie beispielsweise 1-Phenyl-3-methylpyrazol-5-on,
- Indolderivate wie beispielsweise 4-Hydroxyindol, 6-Hydroxyindol und 7-Hydroxyindol,
- Methylendioxybenzolderivate wie beispielsweise 1-Hydroxy-3,4-methylendioxybenzol, 1-Amino-3,4-methylendioxybenzol und 1-(2'-Hydroxyethyl)-amino-3,4-methylendioxybenzol.

Besonders geeignete Kupplerkomponenten sind 1-Naphthol, 1,5-, 2,7- und 1,7-Dihydroxynaphthalin, 3-Aminophenol, 5-Amino-2-methylphenol, 2-Amino-3-hydroxypyridin, Resorcin, 4-Chlorresorcin, 2-Chlor-6-methyl-3-aminophenol, 2-Methylresorcin, 5-Methylresorcin, 2,5-Dimethylresorcin und 2,6-Dihydroxy-3,4-dimethylpyridin.
Direktziehende Farbstoffe sind üblicherweise Nitrophenylendiamine, Nitroaminophenole, Azofarbstoffe, Anthrachinone oder Indophenole. Besonders geeignete direktziehende Farbstoffe sind die unter den internationalen Bezeichnungen bzw. Handelsnamen HC Yellow 2, HC Yellow 4, HC Yellow 5, HC Yellow 6, Basic Yellow 57, Disperse Orange 3, HC Red 3, HC Red BN, Basic Red 76, HC Blue 2, HC Blue 12, Disperse Blue 3, Basic Blue 99, HC Violet 1, Disperse Violet 1, Disperse Violet 4, Disperse Black 9, Basic Brown 16 und Basic Brown 17 bekannten Verbindungen sowie 1,4-Bis-(β-hydroxyethyl)-amino-2-nitrobenzol, 4-Amino-2-nitrodiphenylamin-2'-carbonsäure, 6-Nitro-1,2,3,4-tetrahydrochinoxalin, Hydroxyethyl-2-nitro-toluidin, Pikraminsäure, 2-Amino-6-chloro-4-nitrophenol, 4-Ethylamino-3-nitrobenzoesäure und 2-Chloro-6-ethylamino-1-hydroxy-4-nitrobenzol.

In der Natur vorkommende direktziehende Farbstoffe sind beispielsweise die in Henna rot, Henna neutral, Henna schwarz, Kamillenblüte, Sandelholz, schwarzen Tee, Faulbaumrinde, Salbei, Blauholz, Krappwurzel, Catechu, Sedre und Alkannawurzel enthaltenen färbenden Verbindungen.

Es ist nicht erforderlich, dass die Oxidationsfarbstoffvorprodukte oder die direktziehenden Farbstoffe jeweils einheitliche Verbindungen darstellen. Vielmehr können in den erfindungsgemäßen Haarfärbemitteln, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z. B. toxikologischen, ausgeschlossen werden müssen.

Bezüglich der in den erfindungsgemäßen Haarfärbe- und -tönungsmitteln einsetzbaren Farbstoffe wird weiterhin ausdrücklich auf die Monographie Ch. Zviak, The Science of Hair Care, Kapitel 7 (Seiten 248-250; direktziehende Farbstoffe) sowie Kapitel 8, Seiten 264-267; Oxidationsfarbstoffvorprodukte), erschienen als Band 7 der Reihe "Dermatology" (Hrg.: Ch., Culnan und H. Maibach), Verlag Marcel Dekker Inc., New York, Basel, 1986, sowie das "Europäische Inventar der Kosmetik-Rohstoffe", herausgegeben von der Europäischen Gemeinschaft, erhältlich in Diskettenform vom Bundesverband Deutscher Industrie- und Handelsunternehmen für Arzneimittel, Reformwaren und Körperpflegemittel e.V., Mannheim, Bezug genommen.
Als Vorstufen naturanaloger Farbstoffe werden beispielsweise Indole und Indoline sowie deren physiologisch verträgliche Salze verwendet. Bevorzugt werden solche Indole und Indoline eingesetzt, die mindestens eine Hydroxy- oder Aminogruppe, bevorzugt als Substituent am Sechsring, aufweisen. Diese Gruppen können weitere Substituenten tragen, z. B. in Form einer Veretherung oder Veresterung der Hydroxygruppe oder eine Alkylierung der Aminogruppe. Besonders vorteilhafte Eigenschaften haben 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin, 5,6-Dihydroxyindolin-2-carbonsäure, 6-Hydroxyindolin, 6-Aminoindolin und 4-Aminoindolin sowie 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 5,6-Dihydroxyindol-2-carbonsäure, 6-Hydroxyindol, 6-Aminoindol und 4-Aminoindol.

Besonders hervorzuheben sind innerhalb dieser Gruppe N-Methyl-5,6-dihydroxyindolin, N-Ethyl-5,6-dihydroxyindolin, N-Propyl-5,6-dihydroxyindolin, N-Butyl-5,6-dihydroxyindolin und insbesondere das 5,6-Dihydroxyindolin sowie N-Methyl-5,6-dihydroxyindol, N-Ethyl-5,6-dihydroxyindol, N-Propyl-5,6-dihydroxyindol, N-Butyl-5,6-dihydroxyindol sowie insbesondere das 5,6-Dihydroxyindol.

Die Indolin- und Indol-Derivate in den im Rahmen des erfindungsgemäßen Verfahrens eingesetzten Färbemitteln sowohl als freie Basen als auch in Form ihrer physiologisch verträglichen Salze mit anorganischen oder organischen Säuren, z. B. der Hydrochloride, der Sulfate und Hydrobromide, eingesetzt werden.

Bei der Verwendung von Farbstoff-Vorstufen vom Indolin- oder Indol-Typ kann es bevorzugt sein, diese zusammen mit mindestens einer Aminosäure und/oder mindestens einem Oligopeptid einzusetzen. Bevorzugte Aminosäuren sind Aminocarbonsäuren, insbesondere α-Aminocarbonsäuren und ω-Aminocarbonsäuren. Unter den α-Aminocarbonsäuren sind wiederum Arginin, Lysin, Ornithin und Histidin besonders bevorzugt. Eine ganz besonders bevorzugte Aminosäure ist Arginin, insbesondere in freier Form, aber auch als Hydrochlorid eingesetzt.

Haarfärbemittel, insbesondere wenn die Ausfärbung oxidativ, sei es mit Luftsauerstoff oder anderen Oxidationsmitteln wie Wasserstoffperoxid, erfolgt, werden üblicherweise schwach sauer bis alkalisch, d. h. auf pH-Werte im Bereich von etwa 5 bis 11, eingestellt. Zu diesem Zweck enthalten die Färbemittel Alkalisierungsmittel, üblicherweise Alkali- oder Erdalkalihydroxide, Ammoniak oder organische Amine. Bevorzugte Alkalisierungsmittel sind Monoethanolamin, Monoisopropanolamin, 2-Amino-2-methyl-propanol, 2-Amino-2-methyl-1,3-propandiol, 2-Amino-2-ethyl-1,3-propandiol, 2-Amino-2-methylbutanol und Triethanolamin sowie Alkali- und Erdalkalimetallhydroxide. Insbesondere Monoethanolamin, Triethanolamin sowie 2-Amino-2-methyl-propanol und 2-Amino-2-methyl-1,3-propandiol sind im Rahmen dieser Gruppe bevorzugt. Auch die Verwendung von ω-Aminosäuren wie ω-Aminocapronsäure als Alkalisierungsmittel ist möglich.

Erfolgt die Ausbildung der eigentlichen Haarfarben im Rahmen eines oxidativen Prozesses, so können übliche Oxidationsmittel, wie insbesondere Wasserstoffperoxid oder dessen Anlagerungsprodukte an Harnstoff, Melamin oder Natriumborat verwendet werden. Die Oxidation mit Luftsauerstoff als einzigem Oxidationsmittel kann allerdings bevorzugt sein. Weiterhin ist es möglich, die Oxidation mit Hilfe von Enzymen durchzufiihren, wobei die Enzyme sowohl zur Erzeugung von oxidierenden Per-Verbindungen eingesetzt werden als auch zur Verstärkung der Wirkung einer geringen Menge vorhandener Oxidationsmittel, oder auch Enzyme die Elektronen aus geeigneten Entwicklerkomponenten (Reduktionsmittel) auf Luftsauerstoff übertragen. Bevorzugt sind dabei Oxidasen wie Tyrosinase, Ascorbatoxidase und Laccase aber auch Glucoseoxidase, Uricase oder Pyruvatoxidase. Weiterhin sei das Vorgehen genannt, die Wirkung geringer Mengen (z. B. 1% und weniger, bezogen auf das gesamte Mittel) Wasserstoffperoxid durch Peroxidasen zu verstärken.

Zweckmäßigerweise wird die Zubereitung des Oxidationsmittels dann unmittelbar vor dem Färben der Haare mit der Zubereitung mit den Farbstoffvorprodukten vermischt. Das dabei entstehende gebrauchsfertige Haarfärbepräparat sollte bevorzugt einen pH-Wert im Bereich von 6 bis 10 aufweisen. Besonders bevorzugt ist die Anwendung der Haarfärbemittel in einem schwach alkalischen Milieu. Die Anwendungstemperaturen können in einem Bereich zwischen 15 und 40 °C, bevorzugt bei der Temperatur der Kopfhaut, liegen. Nach einer Einwirkungszeit von ca. 5 bis 45, insbesondere 15 bis 30, Minuten wird das Haarfärbemittel durch Ausspülen von dem zu färbenden Haar entfernt. Das Nachwaschen mit einem Shampoo entfällt, wenn ein stark tensidhaltiger Träger, z. B. ein Färbeshampoo, verwendet wurde.

Insbesondere bei schwer färbbarem Haar kann die Zubereitung mit den Farbstoffvorprodukten ohne vorherige Vermischung mit der Oxidationskomponente auf das Haar aufgebracht werden. Nach einer Einwirkdauer von 20 bis 30 Minuten wird dann - gegebenenfalls nach einer Zwischenspülung - die Oxidationskomponente aufgebracht. Nach einer weiteren Einwirkdauer von 10 bis 20 Minuten wird dann gespült und gewünschtenfalls nachshampooniert. Bei dieser Ausführungsform wird gemäß einer ersten Variante, bei der das vorherige Aufbringen der Farbstoffvorprodukte eine bessere Penetration in das Haar bewirken soll, das entsprechende Mittel auf einen pH-Wert von etwa 4 bis 7 eingestellt. Gemäß einer zweiten Variante wird zunächst eine Luftoxidation angestrebt, wobei das aufgebrachte Mittel bevorzugt einen pH-Wert von 7 bis 10 aufweist. Bei der anschließenden beschleunigten Nachoxidation kann die Verwendung von sauer eingestellten Peroxidisulfat-Lösungen als Oxidationsmittel bevorzugt sein.

Weiterhin kann die Ausbildung der Färbung dadurch unterstützt und gesteigert werden, dass dem Mittel bestimmte Metallionen zugesetzt werden. Solche Metallionen sind beispielsweise $Zn^{2+}$, $Cu^{2+}$, $Fe^{2+}$, $Fe^{3+}$, $Mn^{2+}$, $Mn^{4+}$, $Li^{+}$, $Mg^{2+}$, $Ca^{2+}$ und $Al^{3+}$. Besonders geeignet sind dabei $Zn^{2+}$, $Cu^{2+}$ und $Mn^{2+}$. Die Metallionen können prinzipiell in der Form eines beliebigen, physiologisch verträglichen Salzes eingesetzt werden. Bevorzugte Salze sind die Acetate, Sulfate, Halogenide, Lactate und Tartrate. Durch Verwendung dieser Metallsalze kann sowohl die Ausbildung der Färbung beschleunigt als auch die Farbnuance gezielt beeinflusst werden.

Weitere fakultative Inhaltsstoffe der erfindungsgemäßen Mittel sind

- nichtionische Polymere wie beispielsweise Vinylpyrrolidon/Vinylacrylat-Copolymere, Polyvinylpyrrolidon und Vinylpyrrolidon/Vinylacetat-Copolymere,
- anionische Polymere, wie Polyacryl- und Polymethacrylsäuren in Form ihrer Copolymere mit Acrylsäure- und Methacrylsäureestern und -amiden, Polyoxycarbonsäuren, wie Polyketo- und Polyaldehydocarbonsäuren und deren Salze, sowie Polymere und Copolymere der Crotonsäure mit Estern und Amiden der Acryl- und der Methacrylsäure, wie Vinylacetat-Crotonsäure- und Vinylacetat-Vinylpropionat-Crotonsäure-Copolymere,
- Strukturanten wie Glucose und Maleinsäure,
- haarkonditionierende Verbindungen wie Phospholipide, beispielsweise Sojalecithin, Ei-Lecithin und Kephaline,
- Parfümöle, insbesondere solche mit der Duftnote einer Frucht, wie beispielsweise von Apfel, Birne, Erdbeere, Pfirsich, Aprikose, Ananas, Banane, Kirsche, Kiwi, Mango, Kokos, Mandel, Grapefruit, Maracuja, Mandarine und

Melone, oder der Duftnote eines Genussmittels, wie beispielsweise von Tabak, Cola, Kaugummi, Guarana, Schokolade, Kakao, Vanille, Sarsaparilla, Pfefferminze und Rum. Dimethylisosorbid und Cyclodextrine,

- Lösungsvermittler, wie Ethanol, Isopropanol, Ethylenglykol, Propylenglykol, Glycerin, Diethylenglykol und ethoxylierte Triglyceride,
- Farbstoffe,
- Antischuppenwirkstoffe wie Climbazol, Piroctone Olamine und Zink Omadine,
- Wirkstoffe wie Bisabolol, Allantoin, Panthenol, Niacinmid, Tocopherol und Pflanzenextrakte,
- Lichtschutzmittel,
- Konsistenzgeber wie Zuckerester, Polyolester oder Polyolalkylether,
- Fette und Wachse, wie Walrat, Bienenwachs, Montanwachs, Paraffine, Ester, Glyceride und Fettalkohole,
- Fettsäurealkanolamide,
- Komplexbildner wie EDTA, NTA, β-Alanindiessigsäure und Phosphonsäuren,
- Quell- und Penetrationsstoffe wie PCA, Glycerin, Propylenglykolmonoethylether, Carbonate, Hydrogencarbonate, Guanidine, Harnstoffe sowie primäre, sekundäre und tertiäre Phosphate,
- Trübungsmittel wie Latex oder Styrol/Acrylamid-Copolymere,
- Perlglanzmittel wie Ethylenglykolmono- und -distearat oder PEG-3-distearat,
- Weißpigmente
- Reduktionsmittel wie z.B. Thioglykolsäure und deren Derivate, Thiomilchsäure, Cysteamin, Thioäpfelsäure und α-Mercaptoethansulfonsäure,
- Oxidationsmittel wie Wasserstoffperoxid, Kaliumbromat und Natriumbromat,
- Treibmittel wie Propan-Butan-Gemische, $N_2O$ Dimethylether, $CO_2$ und Luft sowie
- Antioxidantien,
- Bitterstoffe, wie beispielsweise Denatonium Benzoate,
- Konservierungsmittel.

Hinsichtlich der Art, gemäß der erfindungsgemäße verwendete Wirkstoff beziehungsweise die erfindungsgemäßen Wirkstoffkombinationen auf das Haar und/oder auf die Haut aufgebracht werden, bestehen keine prinzipiellen Einschränkungen. Als Konfektionierung dieser Zubereitungen sind beispielsweise Cremes, Lotionen, Lösungen, Wässer, Emulsionen wie W/O-, O/W-, PIT-Emulsionen (Emulsionen nach der Lehre der Phaseninversion, PIT genannt), Mikroemulsionen und multiple Emulsionen, Nanoemulsionen, grobe, instabile, ein oder mehrphasige Schüttelmixturen, Gele, Sprays, Aerosole und Schaumaerosole geeignet. Diese werden in der Regel auf wässriger oder wässrig-alkoholischer Basis formuliert. Als alkoholische Komponente kommen dabei niedere Alkanole sowie Polyole wie Propylenglykol und Glycerin zum Einsatz. Ethanol und Isopropanol sind bevorzugte Alkohole. Wasser und Alkohol können in der wässrig alkoholischen Basis in einem Gewichtsverhältnis von 1 : 10 bis 10 : 1 vorliegen. Wasser sowie wässrig-alkoholische Mischungen, die bis zu 50 Gew.-%, insbesondere bis zu 25 Gew.-%, Alkohol, bezogen auf das Gemisch Alkohol/Wasser, enthalten, können erfindungsgemäß bevorzugte Grundlagen sein. Der pH-Wert dieser Zubereitungen kann prinzipiell bei Werten von 2-11 liegen. Er liegt bevorzugt zwischen 2 und 7, wobei Werte von 3 bis 6 besonders bevorzugt sind. Zur Einstellung dieses pH-Wertes kann praktisch jede für kosmetische Zwecke verwendbare Säure oder Base verwendet werden. Üblicherweise werden als Säuren Genusssäuren verwendet. Unter Genusssäuren werden solche Säuren verstanden, die im Rahmen der üblichen Nahrungsaufnahme aufgenommen werden und positive Auswirkungen auf den menschlichen Organismus haben. Genusssäuren sind beispielsweise Essigsäure, Milchsäure, Weinsäure, Zitronensäure, Äpfelsäure, Ascorbinsäure und Gluconsäure. Im Rahmen der Erfindung ist die Verwendung von Zitronensäure und Milchsäure besonders bevorzugt. Bevorzugte Basen sind Ammoniak, Alkalihydroxide, Monoethanolamin, Triethanolamin sowie N,N,N',N'-Tetrakis-(2-hydroxypropyl)-ethylendiamin.

In einer besonderen Ausführungsform der erfindungsgemäßen Mittel kann es bevorzugt sein, wenn die Mittel als Mikroemulsion vorliegen. Unter Mikroemulsionen werden im Rahmen der Erfindung ebenfalls sogenannte "PIT"-Emulsionen verstanden. Bei diesen Emulsionen handelt es sich im Prinzip um Systeme mit den 3 Komponenten Wasser, Öl und Emulgator, die bei Raumtemperatur als Öl-in-Wasser(O/W)-Emulsion vorliegen. Beim Erwärmen dieser Systeme bilden sich in einem bestimmten Temperaturbereich (üblicherweise als Phaseninversiontemperatur oder "PIT" bezeichnet) Mikroemulsionen aus, die sich bei weiterer Erwärmung in Wasser-in-Öl(W/O)-Emulsionen umwandeln. Bei anschließendem Abkühlen werden wieder O/W-Emulsionen gebildet, die aber auch bei Raumtemperatur als Mikroemulsionen mit einem mittleren Teilchendurchmesser von kleiner als 400 nm, insbesondere mit einem Teilchendurchmesser von etwa 100-300 nm, vorliegen. Einzelheiten bezüglich dieser sehr stabilen, niedrigviskosen Systeme, für die sich die Bezeichnung "PIT-Emulsionen" allgemein durchgesetzt hat, sind einer Vielzahl von Druckschriften zu entnehmen, für die stellvertretend die Veröffentlichungen in Angew. Chem. 97, 655-669 (1985) und Adv. Colloid Interface Sci 58, 119-149 (1995) genannt werden.

Erfindungsgemäß können solche Mikro- oder "PIT"-Emulsionen bevorzugt sein, die einen mittleren Teilchendurchmesser von etwa 200 nm aufweisen.

Die Herstellung der erfindungsgemäßen Mikroemulsionen kann beispielsweise in der Art erfolgen, dass zunächst die Phaseninversionstemperatur des Systems bestimmt wird, indem man eine Probe der auf übliche Weise hergestellten Emulsion erhitzt und unter Verwendung eines Leitfähigkeitsmessgerätes die Temperatur bestimmt, bei der die Leitfahigkeit stark abnimmt. Die Abnahme der spezifischen Leitfähigkeit der zunächst vorhandenen O/W-Emulsion nimmt dabei in der Regel über einen Temperaturbereich von 2 bis 8 °C von ursprünglich mehr als 1 mS/cm auf Werte unterhalb von 0,1 mS/cm ab. Dieser Temperaturbereich entspricht dann dem Phaseninversions-Temperaturbereich. Nachdem somit der Phaseninversions-Temperaturbereich bekannt ist, kann man die zunächst wie üblich hergestellte Emulsion aus Ölkomponente, nichtionogenem Emulgator, zumindest Teilen des Wassers sowie gegebenenfalls weiteren Komponenten auf eine Temperatur erhitzen, die innerhalb oder oberhalb des Phaseninversions-Temperaturbereiches liegt, sodann abkühlen und gegebenenfalls weitere Komponenten sowie das restliche Wasser hinzufügen. Alternativ kann auch die Herstellung der Mikroemulsion direkt bei einer Temperatur erfolgen, die innerhalb oder oberhalb des Phaseninversions-Temperaturbereiches liegt. Die so hergestellte Mikroemulsion wird dann auf eine Temperatur unterhalb des Phaseninversions-Temperaturbereiches, üblicherweise Raumtemperatur, abgekühlt.
Die erfindungsgemäßen Mittel können nach einer Einwirkzeit wieder von dem Haar entfernt werden oder sie können auch auf dem Haar verbleiben. Unter auf dem Haar verbleibend werden erfindungsgemäß solche Zubereitungen verstanden, die nicht im Rahmen der Behandlung nach einem Zeitraum von wenigen Sekunden bis zu einer Stunde mit Hilfe von Wasser oder einer wässrigen Lösung wieder aus dem Haar ausgespült werden. Vielmehr verbleiben die Zubereitungen bis zur nächsten Wäsche auf dem Haar.
Die folgenden Beispiele sollen die Erfindung näher erläutern.

**Beispiele**

*I. Nachweise der Wirksamkeit von Ectoin in Haarreinigungs- und Haarbehandlungsmitteln*

I.1 Untersuchung der Fönschutzeisgenschaften von Ectoin

Es sollte die Wirksamkeit einer wässrigen Zubereitung von Ectoin zur Verringerung der Hitzeschädigung des Haares geprüft werden. Als Referenz diente eine Messung mit Wasser als Behandlungslösung.

Haarbehandlung

1. Vermessung der Haarquerschnitte im nassen Zustand.
2. Bestimmung der Spannungswerte, Gradienten, E-Moduli und Arbeitswerte der nassen Haare für 1% Haardehnung. Die Messung wurde mit dem Vollautomat MIT 670 der Firma Dia Stron durchgeführt.
3. 50 x 80°C Fönen, dazwischen 5 Minuten Behandlung mit der jeweiligen Behandlungslösung (Wasser bzw. wässrige 1%ige Ectoinlösung).
4. Zug-Dehnungsmessung wie in Punkt 2.

Methode

Zerstörungsfreie Materialprüfung durch Bestimmung des Hook'schen Bereiches (1% Dehnung) der Haare im unbehandelten und behandelten Zustand. Durch dieses Verfahren ist es möglich, eine Differenzmessung am Einzelhaar mit einem t-Test für Paare durchzuführen. Es wurden für die Messreihen jeweils 40 Einzelhaare verwendet.
Test-Haarproben: Natural dark brown code #6634 der Firma Alkino

Tabelle 1: Überprüfung der Schädigung durch Fönhitze mit Wasser als Behandlungslösung

| Haarquerschnitts-fläche [$\mu m^2$] | Gradient [N/mm] | Elastizitäts- modul [$N/m^2$] | Arbeit bei 1% Dehnung [J] | Spannungswert bei 1% Dehnung [$N/\mu m^2$] |
|---|---|---|---|---|
| **Unbehandeltes Haar:** | | | | |
| 3.99E+03 | 3,15E-01 | 2,55E+09 | 1,53E-05 | 2,54E-05 |
| **50x80°C Fönen / Zwischenbehandlung mit Wasser:** | | | | |
| 3,99E+03 | 2,77E-01 | 2,26E+09 | 1,35E-05 | 2,27E-05 |

Tabelle fortgesetzt

| 50x80°C Fönen / Zwischenbehandlung mit Wasser: | | | | |
|---|---|---|---|---|
| Stat. Vergleich Test: Zweiseitig, paarweise unbeh./beh. | 99,95% | 99,85% | 99,89% | 99,83% |

Tabelle 2: 1%ige Ectoinlösung als Behandlungslösung

| Haarquerschnitts-fläche [$\mu m^2$] | Gradient [N/mm] | Elastizitätsmodul [$N/m^2$] | Arbeit bei 1% Dehnung [J] | Spannungswert bei 1% Dehnung [$N/\mu m^2$] |
|---|---|---|---|---|
| **Unbehandeltes Haar:** | | | | |
| 3.77E+03 | 2,66E-01 | 2,20E+09 | 1,27E-05 | 2,24E-05 |
| **50x80°C Fönen / Zwischenbehandlung mit 1%iger Ectoinlösung:** | | | | |
| 3,77E+03 | 2,53E-01 | 2,09E+09 | 1,22E-05 | 2,11E-05 |
| Stat. Vergleich Test: Zweiseitig, paarweise unbeh./beh. | 87,10% | 90,91% | 79,67% | 92,98% |

Die o. g. Prozentwerte beim statistischen t-Test geben die prozentuale Wahrscheinlichkeit an, mit der die Messreihen im zweiseitigen, paarweisen Vergleich unterschieden sind. Bei einem Wert >95% sind die Messreihen als unterschieden anzusehen. Bei einem Wert zwischen 90 und 95% ist eine deutliche Tendenz zu sehen.

Fazit

Die Behandlung der Haare mit 1%iger Ectoinlösung (pH-Wert 7) schützt die Haare vor der Fönhitze.

I.2 Feuchtemessung an mit Ectoinlösung behandelten Haarsträhnen

Es wurde das RonaCareTM Ectoin (Fa. Merck, 1%ige wässrigeLösung) auf eine feuchtigkeitsbeeinflussende Wirkung am europäischen Haar untersucht. Als Referenz diente eine Messung mit Wasser als Testlösung.

Haarbehandlung

Es wurden jeweils 48 Haarsträhnen vermessen, die in der folgenden Weise manuell vorbehandelt wurden:

1. unbehandelte Haare
2. klimatisieren (24 Stunden bei 25°C und 40% relativer Feuchtigkeit.
3. Nullwertmessung.
4. 10 Minuten in die jeweilige Testlösung tauchen (nicht ausspülen)
5. Haare 1 Stunde bei 80°C mit dem Umlufltrockner trocknen.
6. 10-fache Wiederholung der Schritte 4 bis 6.

Methode

Jede Haarsträhne wurde sowohl im unbehandelten, als auch im behandelten Zustand hinsichtlich der Feuchtigkeit (Mikrowellentechnik) vermessen.

Die Mittelwertdifferenzen der Vorher-/Nachher-Messungen sind der nachfolgenden Tabelle zu entnehmen:

| | **Relative Differenzfeuchte Vorher / Nachher** |
|---|---|
| **1%ige Ectoinlösung** | +0,010 |

Tabelle fortgesetzt

| | Relative Differenzfeuchte Vorher / Nachher |
|---|---|
| **Wasser** | -0,005 |
| **unbehandelte Haarsträhne**n | +0,000 |

Je höher die Differenzfeuchte bei der Mikrowellenmethode (Differenz vor und nach der Behandlung) gegenüber den unbehandelten Haaren, desto mehr Feuchtigkeit wurde im Haar nach der Konditionierungsphase eingelagert.

Fazit

Die Haarbehandlung mit einer 1%igen Ectoinlösung zeigt feuchtigkeitsbindende Eigenschaften gegenüber den unbehandelten und den nur mit Wasser behandelten Haaren.

I.3 Bestimmung der Widerstandskraft von mit Ectoin behandelten Haaren anhand des Ermüdungsbruches von Einzelhaaren durch einen Reib-Biege-Test

Es wurde untersucht, inwieweit Ectoin die Widerstandskraft von zweifach dauergewellten Einzelhaaren bei einer mechanischen Kombinationsbelastung durch Reiben und Biegen beeinflusst.
Die Messungen wurden mit einem Reib-Biege-Testgerät der Firma Textechno, Herbert Stein GmbH&Co. KG Mönchengladbach unter den folgenden Meßbedingungen durchgeführt:

- 65% relative Luftfeuchtigkeit,
- 22°C,
- Einstellungen am Prüfgerät:
    - Drahtdurchmesser: 0,3 mm
    - Frequenz: 1 Hz
    - Amplitude(Hub): 2 mm
    - Belastungsmasse: 20 g
    - Länge des jeweiligen Einzelhaarsegments: 10 mm.

Die verwendeten Haare wurden der Probandin direkt an der Kopfhaut abgeschnitten. Zur Messung kamen die ersten 6 cm im wurzelnahen Bereich zur Anwendung. Die 6 cm langen Haarstücke wurden in weitere 3 Segmente zerteilt, zyklisch vertauscht und auf drei Messreihen verteilt:

1. unbehandelte Haare
2. 2x kaltgewellte Haare (Kaltwelle: 7% Thioglycolsäure (INCI-Bezeichnung: Thioglycolic Acid), 0,3% Turpinal SL (Etidronsäure; INCI-Bezeichnung: Etidronic Acid), 3,5% $(NH_4)_2CO_3$, pH-Wert: 8,4; Fixierung: 2% H2O2, 1% Turpinal SL, pH-Wert-Einstellung mit Ammoniak auf pH 4,0)
3. Kombinationsbehandlung von 2 Kaltwellen (vgl. Punkt 2) und 3 Ectoin-Applikationen

Haarbehandlung

1. 15 Minuten Behandlung mit einer 2%igen Ectoinlösung.
2. Die Haare werden 10 Sekunden ausgespült.
3. 30-minütige Applikation der Kaltwelle, gefolgt von einem Ausspüldurchgang von 5 Minuten.
4. 10-minütige Applikation der Fixierlösung, gefolgt von einem Ausspüldurchgang von 5 Minuten.
5. 15-minütige Behandlung mit 2%iger Ectoinlösung.
6. 10-minütiger Ausspülvorgang.
7. Die Punkte 3 bis 6 werden wiederholt.

Methode

Es wurde die Anzahl der Zyklen ermittelt, die bis zum Haarbruch durchlaufen wurden. Der nachfolgenden Tabelle sind die Mittelwerte der Ergebnisse aus drei Messreihen mit je 21 Messungen zu entnehmen.

Zusätzlich wurde ein statistischer Vergleichstest durchgeführt (Student-t-Test, zweiseitig, paarweise). Demnach sind die 2x kaltgewellten Haare zu den Ectoin / kaltgewellten Haaren mit einer statistischen Wahrscheinlichkeit von ca. 94% unterschieden wurden (p-Wert: 0,05972).

| | **unbehandelt** | **2xKaltwelle** | **Ectoin/Kaltwelle** |
|---|---|---|---|
| **Mittelwert** | **795** | **505** | **661** |
| Standardabweichung | 604 | 295 | 380 |
| VK (Variationskoeffizient in %) $\frac{\text{Standardabweichung x 100}}{\text{Mittelwert}}$ | 76,06 | 58,46 | 57,43 |

Fazit

Die Messwerte zeigen, dass die Behandlung mit Ectoin zu einer Erhöhung der Widerstandskraft der Haare führt. Die mittlere "Überlebensrate" wird von 505 Zyklen auf 661 Zyklen erhöht.

1.3 Goniotropische Glanzmessungen zur Bestimmung des Haarglanzes von mit Ectoin behandelten Haaren

Für die Messungen wurden Euro-Naturhaar-Strähnen ("Klebetressen dicht", der Firma Kerling, 22 cm lang, 1,5 cm breit, ca. 2 g schwer) verwendet. Diese wurden zunächst mit einer 14%igen Texapon® NSO-Lösung (pH 6,5) gewaschen (15 Minuten) und anschließend mit Leitungswasser ausgespült.
Danach wurden die Haare mit einem gleichmäßigen Fettsäurefilm glanzmindernd vorbehandelt, indem die Haartressen zunächst für 20 Minuten in etwa 1 Liter einer 1%igen Lösung einer handelsüblichen Kernseife (Dalli-Werke, 52220 Stolberg, UBA02500166, Pflegekernseife aus reinem Pflanzenöl, unparfümiert, gelöst in demineralisiertem Wasser) bei 45°C belassen und anschließend 2x in je 2 Liter demineralisiertem Wasser gespült wurden.

Die Haarsträhnen wurden daraufhin unter konditionierenden Bedingungen (25°C, 40% relative Feuchtigkeit) über Nacht flach liegend getrocknet.

Am Folgetag wurden die vorbehandelten Haartressen (5 Haartressen pro Testprodukt) in der Glanzapparatur vermessen. Dabei erhält man bestimmte Werte als Blindpunkte, die daher ohne Einheit [o.E.] angegeben sind.

Als Vergleichsstandard diente eine Texapon-NSO-Lösung (INCI: Sodium Laureth Sulfate) mit einem Gehalt von 12% Aktivsubstanz.

Die Messung erfolgte derart, dass die Haartressen mit 2g des jeweiligen Testproduktes (1%ige Ectoinlösung in Texapon NSO-Lösung sowie Texapon NSO-Lösung) gewaschen wurden. Nach 5 Minuten Einwirkungszeit wurden die Haartressen 1 Minute lang mit Leitungswasser ausgewaschen und anschließend nochmals unter konditionierenden Bedingungen flach liegend getrocknet. Nach dieser Konditionierung erfolgte erneut eine Glanzmessung.

Die Ergebnisse der Glanzmessungen, die der nachfolgenden Tabelle zu entnehmen sind, setzen sich aus Mittelwertdifferenzen zusammen, die durch folgende Schritte definiert wurden:

- jede Haartresse wurde sowohl im unbehandelten (glanzmindernd vorbehandelt) als auch im jeweils behandelten Zustand jeweils 5x bezüglich des Glanzes vermessen. Daraus ergibt sich eine Anzahl von 25 Messungen pro Wirknachweis.
- zur Beurteilung des Glanzverhaltens wurden die fünf Messreihen je Haartresse zu einem Mittelwert zusammengefasst, so dass abschließend pro Wirknachweis 10 Mittelwert-Messreihen (5x unbehandelt gegen 5x behandelt) gegeneinander verglichen wurden.
- für die statistische Auswertung wurde die Teststatistik eines Student'schen t-Tests (abhängig, zweiseitiger Test, gepaart) angewendet. Als Prüfgröße wurden hierzu die Maxima der 10 Mittelwert-Messreihen verwendet und zu einem Vorher-Nachher-Wert gemittelt

Glanzintensitätsmessung im Vergleich zum mattierten Haar nach der 1. Behandlung:

| Glanzmindernd vorbehandelt und 1x gewaschen mit... | Glanzintensitätsänderung gegenüber mattierten Haaren [ohne Einheit] | Glanzintensitätsänderung gegenüber mattierten Haaren [%] | Statistik (p-Wert) | Glanzfaktor gegenüber Texapon-NSO-Lösung |
|---|---|---|---|---|
| **1%iger Ectoinlösung** in Texapon-NSO-Lösung (12% Aktivsubstanz) | 0,0126 | 2,0 | 0,036 | 1,3 |
| **Texapon-NSO-Lösung (12% Aktivsubstanz)** | 0,0094 | 1,5 | 0,043 | --- |

Fazit

Für das mit Ectoinlösung behandelte Haar konnte eine signifikante Glanzsteigerung nachgewiesen werden. Im Vergleich zum mit Texaponlösung (WAS 12%) behandelten Haar konnte für die "Ectoin-Rezeptur" ein Glanzfaktor von 1,3 ermittelt werden.
Der Glanzfaktor stellt einen relativen Wert dar, da das jeweilige Messergebnis auf die jeweilig mitgetestete Texaponlösung bezogen ist.

*I. Anwendungsbeispiele*

Alle Angaben beziehen sich, sofern nicht anders angegeben, auf Gewichtsprozent.

**Fönlotion**

| | |
|---|---|
| Luviskol® VA 64 | 1,0 |
| Luviquat® FC 905 | 0,2 |
| Ethanol | 30,0 |
| Ectoin (AS) | 0,1 |
| Wasser und Milchsäure bis pH=5,5 | ad 100,0 |

**Fönwell-Lotion**

| | |
|---|---|
| Luviflex® D430I | 1,5 |
| Dehyquart® A | 0,2 |
| Ethanol | 20,0 |
| Ectoin (AS) | 0,2 |
| Wasser und Citronensäure bis pH=6 | ad 100,0 |

**Fönwell-Festiger**

| | |
|---|---|
| Luviset® CA 66 | 0,7 |
| Aminomethylpropanol | 0,056 |
| Ethanol | 30,0 |
| Ectoin (AS) | 0,3 |
| Wasser und Milchsäure bis pH=6 | ad 100,0 |

**Haarspülung**

| | |
|---|---|
| Eumulgin® B2 | 0,3 |
| Cetyl/Stearylalkohol | 3,3 |
| Isopropylmyristat | 0,5 |
| Paraffinöl perliquidum 15 cSt DAB 9 | 0,3 |
| Dehyquart® A-CA | 2,0 |
| Ectoin (AS) | 1,0 |
| Citronensäure | 0,4 |
| Phenonip® | 0,8 |
| Wasser | ad 100,0 |

**Haarspülung**

| | |
|---|---|
| Eumulgin® B2 | 0,3 |
| Cetyl/Stearylalkohol | 3,3 |
| Isopropylmyristat | 0,5 |
| Paraffinöl perliquidum 15 cSt. DAB 9 | 0,3 |
| Dehyquart® L, 80 | 2,0 |
| Ectoin (AS) | 0,5 |
| Citronensäure | 0,4 |
| Phenonip® | 0,8 |
| Wasser | ad 100,0 |

**Haarkur (rinse off)**

| | |
|---|---|
| Dehyquart® F75 | 4,0 |
| Cetyl/Stearylalkohol | 4,0 |
| Paraffinöl perliquidum 15 cSt DAB 9 | 1,5 |
| Dehyquart® A-CA | 4,0 |
| Salcare® SC96 | 0,5 |
| Ectoin (AS) | 0,5 |
| Citronensäure | 0,15 |
| Phenonip® | 0,8 |
| Wasser | ad 100,0 |

**Haarkur (rinse off)**

| | |
|---|---|
| Dehyquart® L 80 | 4,0 |
| Cetyl/Stearylalkohol | 6,0 |
| Paraffinöl perliquidum 15 cSt. DAB 9 | 2,0 |
| Rewoquat® W 75 | 2,0 |
| Sepigel® 305 | 0,5 |
| Ectoin (AS) | 0,2 |
| Citronensäure | 0,15 |
| Phenonip® | 0,8 |
| Wasser | ad 100,0 |

**Haarkur (auf dem Haar verbleibend)**

| | |
|---|---|
| Dehyquart® F75 | 0,3 |
| Salcare® SC96 | 5,0 |

Tabelle fortgesetzt

| | |
|---|---|
| Dow Coming® 200 Fluid, 5 cSt. | 1,5 |
| Gafquat® 755N | 1,5 |
| Biodocarb® | 0,8 |
| Ectoin (AS) | 0,2 |
| Parfiimöl | 0,25 |
| Wasser | ad 100,0 |

**Haarkur (auf dem Haar verbleibend)**

| | |
|---|---|
| Sepigel® 305 | 5,0 |
| Dow Corning® Q2-5220, 5 cSt. | 1,5 |
| Genamin® DSAC | 0,3 |
| Phenonip® | 0,8 |
| Ectoin (AS) | 0,2 |
| Parfümöl | 0,25 |
| Wasser | ad 100,0 |

**Shampoo**

| | |
|---|---|
| Natriumlaurethsulfat 25% | 40,0 |
| Citronensäure | 0,5 |
| Natriumbenzoat | 0,5 |
| Dinatriumcocoamphodiacetat | 6,0 |
| Salicylsäure | 0,1 |
| Ectoin (AS) | 0,5 |
| Cetiofl® HE | 0,5 |
| Parfüm | 0,4 |
| NaCl | 0,5 |
| Wasser | ad 100,0 |

**Haarstylinggel**

| | |
|---|---|
| Synthalen® K | 0,6 |
| Neutrol® TE | 0,5 |
| Glycerin DAB 9, 86,5 | 8,0 |
| Ethylalkohol, vergaellt 96 Vol% fls | 30,0 |
| Ectoin (AS) | 0,2 |
| Polyethylenglycol | 2,0 |
| PVP/VA W-635 | 6,5 |
| Cremophor® RH40 | 1,0 |
| Parfüm | 0,5 |
| Entsalztes Wasser | ad 100,0 |

**Haarspray**

| | |
|---|---|
| Amphomer® | 3,0 |
| Luviskol® VA37 | 16,0 |
| 2-Amino-2-Methyl-Propanol | 0,6 |
| Isopropylmyristat | 0,12 |
| Ectoin (AS) | 0,5 |
| Parfüm | 0,2 |

Tabelle fortgesetzt

| | |
|---|---|
| Ethylalkohol, vergaellt 96 Vol% fls | 67,5 |
| Entsalztes Wasser | ad 100,0 |

**Haartonic**

| | |
|---|---|
| Panthenol 75 | 0,1 |
| Ectoin (AS) | 0,2 |
| Carbopol® | 0,1 |
| Neutrol® TE | 0,1 |
| Ethylalkohol, vergaellt 96 Vol% fls | 30,0 |
| Entsalztes Wasser | ad 100 |

Es wurden die folgenden Handelsprodukte verwendet

| | |
|---|---|
| Luviskol® VA64: | Vinylpyrrolidon/Vinylacetat-Copolymer (60/40); INCI-Bezeichnung: PVP/VA Copolymer (BASF) |
| Luviquat® FC 905: | Vinylimidazolinium/Vinylpyrrolidon-Copolymer (95/5); INCI-Bezeichnung: Polyquaternium-16 (BASF) |
| Luviflex® D430I: | Vinylpyrrolidon/Vinylacetat/Alkylamino-Acrylat-Copolymer (50%ig in Isopropanol/ Wasser) (BASF) |
| Dehyquart® A: (Dehyquart® A-CA) | Trimethylhexadecylammoniumchlorid INCI-Bezeichnung: Aqua, Cetrimonium Chloride (Cognis) |
| Luviset® CA66: | Vinylacetat-Crotonsäure-Copolymer (90:10); INCI-Bezeichnung: VA/ Crotonates-Copolymer (BASF) |
| Ectoin | 2-Methyl-1,4,5,6-tetrahydro-(4S)-Pyrimidincarbonsäure (Rona (Merck KGaA)) |
| Eumulgin® B2 | Cetylstearylalkohol + 20 EO; INCI-Bezeichnung: Ceteareth-20 (Henkel) |
| Phenonip® | Hydroxybenzoesäuremethylester-Hydroxybenzoesäureethylester-Hydroxybenzoesäurepropylester- Hydroxybenzoesäurebutylester-Phenoxyethanol-Gemisch (ca. 28% Aktivsubstanz); INCI-Bezeichnung: Phenoxyethanol, Methylparaben, Ethylparaben, Propylparaben, Butylparaben) (NIPA) |
| Dehyquart® L 80 | Bis(Cocoethyl)-hydroxyethyl-methylammonium-methosulfat (ca. 76% Aktivsubstanz in Propylenglycol); INCI-Bezeichnung: Dicocoylethyl Hydroxyethylmonium Methosulfate, Propylene Glycol (Henkel) |
| Dehyquart® F75 | Fettalkohol-Methyltriethanolammonium-methylsulfatdialkylester-Gemisch; INCI-Bezeichnung: Distearoylethyl Hydroxyethylmonium Methosulfate, Cetearyl Alcohol) (Henkel) |
| Salcare® SC 96 | INCI-Bezeichnung: Polyquaternium-37, Propylen Dicaprylate/Dicaprate-PPG-1- Tri-deceth-6 (SHC Production Europe GmbH) |
| Rewoquat® W 75 | 1-Methyl-2-nortalgalkyl-3-talgfettsäure-amidoethylimidazolinium-methosulfat (ca. 75% Aktivsubstanz in Propylenglykol); INCI-Bezeichnung: Quaternium-27, Propylene Glycol; (WITCO) |
| Sepigel® 305 | Dodecylalkohol+7EO-Isoparaffin-Polyacrylamid-Zubereitung; INCI-Bezeichnung: Polyacrylamide, C13-14-Isoparaffin, Laureth-7 (Seppic) |
| Dow Corning® 200 Fluid, 5 cSt. | Polydimethylsiloxan; INCI-Bezeichnung: Dimethicone (Dow Corning) |
| Gafquat® 755N | Diemethylaminoethylmethacrylat-Vinylpyrrolidon-Copolymer, mit Diethylsulfat quaterniert (19% Aktivsubstanz in Wasser); INCI-Bezeichnung: Polyquaternium-11 (GAF) |
| Biodocarb® | 3-Iod-2-proinyl-n-butylcarbamat; INCI-Bezeichnung: Iodopropynyl Butylcarbamate (Milker & Grüning) |
| Dow Corning® Q2-5220, 5 cSt. | Silikon-Glykol-Copolymer; INCI-Bezeichnung: Dimethicone Copolyol) (Dow Corning) |

Tabelle fortgesetzt

| | |
|---|---|
| Genamin® DSAC | Dimethyldistearylammoniumchlorid; INCI-Bezeichnung: Distearyldimonium Chloride (Clariant) |
| Cetiol® HE | Kokosmonoglycerid + 7.3 EO; INCI-Bezeichnung: PEG-7 Glyceryl Cocoate (Cognis) |
| Synthalen® K | Polyacrylsäure; INCI-Bezeichnung: Carbomer (Sigma) |
| Neutrol® TE | Tetrakis-(2-hydroxypropyl)-ethylendiamin N,N,N',N'-Edetol; INCI-Bezeichnung: Tetrahydroxypropyl Ethylenediamine (BASF) |
| Cremophor® RH 40 | Glycerinpolyethylenglykoloxystearat-Glycerinethoxylat-Fettsäurepolyethylenglykolester-Polyethylenglykole-Gemisch; INCI-Bezeichnung: PEG-40 Hydrogenated Castor Oil (BASF) |
| Luviskol® VA 37 | Vinylacetat-Vinylpyrrolidon-Copolymer (70:30); INCI-Bezeichnung: PVP/VA Copolymer (BASF) |
| Panthenol 75 | D-Panthenylalkohol; INCI-Bezeichnung: Panthenol (Hoffmann La-Roche) |
| Carbopol® | Acrylpolymer (ca. 30% Aktivsubstanz in Wasser) (BF Goodrich) |

## Patentansprüche

**1.** Verwendung mindestens einer Verbindung der allgemeinen Formel (I) oder (II),

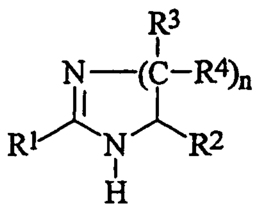

(I)

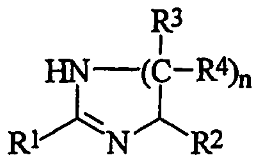

(II)

und/oder eines physiologisch verträglichen Salzes und/oder einer isomeren oder stereoisomeren Form dieser Verbindungen in Haarreinigungs- und/oder Haarbehandlungsmitteln zum Schutz der Haare vor Hitzeschäden, in denen

- $R^1$ steht für ein Wasserstoffatom, einen verzweigten oder unverzweigten $C_1$ - $C_4$-Alkylrest oder einen $C_2$ - $C_4$-Hydroxyalkylrest,
- $R^2$ steht für ein Wasserstoffatom, eine Gruppierung $-COOR^5$ oder eine Gruppierung $-CO(NH)R^5$, wobei $R^5$ für ein Wasserstoffatom, einen $C_1$ - $C_4$-Alkylrest, einen Aminosäurerest, einen Dipeptid- oder einen Tripeptidrest stehen kann,
- $R^3$ und $R^4$ stehen unabhängig voneinander für ein Wasserstoffatom, einen $C_1$ - $C_4$-Alkylrest oder einer der beiden Reste steht für eine Hydroxygruppe und
- n steht für eine ganze Zahl von 1 bis 3.

**2.** Verwendung mindestens einer Verbindung der allgemeinen Formel (I) oder (II) gemäß Anspruch 1 in Haarreinigungs- und/oder Haarbehandlungsmitteln zur Regulierung des Feuchtigkeitsgehalts der Haare.

**3.** Verwendung mindestens einer Verbindung der allgemeinen Formel (I) oder (II) gemäß Anspruch 1 in Haarreinigungs- und/oder Haarbehandlungsmitteln zur Steigerung der Widerstandskraft von Haaren.

**4.** Verwendung mindestens einer Verbindung der allgemeinen Formel (I) oder (II) gemäß Anspruch 1 in Haarreinigungs- und/oder Haarbehandlungsmitteln zur Erhöhung des Haarglanzes.

**5.** Verwendung mindestens einer Verbindung der allgemeinen Formel (I) oder (II) gemäß Anspruch 1 in Haarreinigungs- und/oder Haarbehandlungsmitteln zur Verminderung von Haarbrüchen und Spliss.

**6.** Verwendung gemäß einem der Ansprüche 1 bis 5, **dadurch gekennzeichnet, dass** der Rest $R^1$ für eine Methylgruppe steht.

**7.** Verwendung gemäß einem der Ansprüche 1 bis 6, **dadurch gekennzeichnet, dass** der Rest $R^2$ für die Gruppierung -COOH steht.

**8.** Verwendung gemäß einem der Ansprüche 1 bis 7, **dadurch gekennzeichnet, dass** die Reste $R^3$ und $R^4$ für Wasserstoff stehen.

**9.** Verwendung gemäß einem der Ansprüche 1 bis 8, **dadurch gekennzeichnet, dass** n für die ganze Zahl 2 steht.

**10.** Verwendung gemäß einem der Ansprüche 1 bis 9, **dadurch gekennzeichnet, dass** die Verbindung gemäß Formel (I) oder (II) Ectoin oder eines seiner physiologisch verträglichen Salze ist.

**11.** Verwendung gemäß einem der Ansprüche 1 bis 10, **dadurch gekennzeichnet, dass** das Ectoin oder dessen physiologisch verträgliche Salze in den Haarreinigungs- und/oder Haarbehandlungsmitteln in einer Konzentration von 0,01 bis 2 Gew.-% eingesetzt wird.

**12.** Verwendung gemäß einem der Ansprüche 1 bis 11, **dadurch gekennzeichnet, dass** die Verbindungen gemäß der allgemeinen Formel (I) oder (II) in Haarshampoos, Haarspülungen, Haarkuren, Haar-Tonics, Dauerwell-Fixierlösungen, Haarfärbeshampoos, Haarfestigern, Haarlegemitteln und Haarstyling-Zubereitungen eingesetzt werden.

**Claims**

**1.** Use of at least one compound of the general formula (I) or (II),

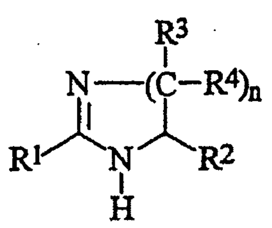

(I)

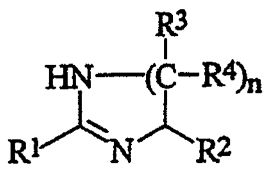

(II)

and/or a physiologically compatible salt and/or an isomeric or stereoisomeric form of these compounds in hair-cleansing and/or hair-treatment compositions for protecting hair against heat damage, in which

- $R^1$ is a hydrogen atom, a branched or unbranched $C_1$-$C_4$-alkyl radical or a $C_2$-$C_4$-hydroxyalkyl radical,
- $R^2$ is a hydrogen atom, a group -$COOR^5$ or a group -CO (NH) $R^5$, where $R^5$ may be a hydrogen atom, a $C_1$-$C_4$-alkyl radical, an amino acid radical, a dipeptide radical or a tripeptide radical,
- $R^3$ and $R^4$, independently of one another, are a hydrogen atom, a $C_1$-$C_4$-alkyl radical or one of the two radicals is a hydroxy group and
- n is an integer from 1 to 3.

**2.** Use of at least one compound of the general formula (I) or (II) according to Claim 1 in hair-cleansing and/or hair-treatment compositions for regulating the moisture content of the hair.

**3.** Use of at least one compound of the general formula (I) or (II) according to Claim 1 in hair-cleansing and/or hair-treatment compositions for increasing the strength of hair.

**4.** Use of at least one compound of the general formula (I) or (II) according to Claim 1 in hair-cleansing and/or hair-treatment compositions for increasing hair shine.

5. Use of at least one compound of the general formula (I) or (II) according to Claim 1 in hair-cleansing and/or hair-treatment compositions for reducing hair breakage and split ends.

6. Use according to one of Claims 1 to 5, **characterized in that** the radical $R^1$ is a methyl group.

7. Use according to one of Claims 1 to 6, **characterized in that** the radical $R^2$ is the group -COOH.

8. Use according to one of Claims 1 to 7, **characterized in that** the radicals $R^3$ and $R^4$ are hydrogen.

9. Use according to one of Claims 1 to 8, **characterized in that** n is the integer 2.

10. Use according to one of Claims 1 to 9, **characterized in that** the compound according to formula (I) or (II) is ectoin or one of its physiologically compatible salts.

11. Use according to one of Claims 1 to 10, **characterized in that** the ectoin or its physiologically compatible salts is used in the hair-cleansing and/or hair-treatment compositions in a concentration of from 0.01 to 2% by weight.

12. Use according to one of Claims 1 to 11, **characterized in that** the compounds according to the general formula (I) or (II) are used in hair shampoos, hair rinses, hair treatments, hair tonics, permanent wave neutralizing solutions, hair colouring shampoos, hair-setting compositions, hair-arranging compositions and hair styling preparations.

**Revendications**

1. Utilisation d’au moins un composé de formule générale (I) ou (II),

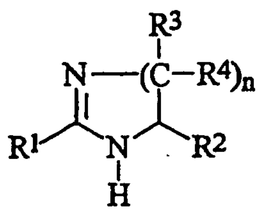

(I)

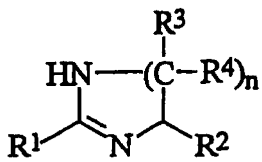

(II)

et/ou d’un sel physiologiquement acceptable et/ou d’une forme isomère ou stéréo-isomère de ces composés dans des agents de nettoyage et/ou de traitement des cheveux pour la protection des cheveux contre des dommages par la chaleur, dans lesquelles

- $R^1$ représente un atome d’hydrogène, un radical alkyle en $C_1$ à $C_4$ ramifié ou non ramifié ou un radical hydroxyalkyle en $C_2$ à $C_4$,
- $R^2$ représente un atome d’hydrogène, un groupement $-COOR^5$ ou un groupement $-CO(NH)R^5$, $R^5$ pouvant représenter un atome d’hydrogène, un radical alkyle en $C_1$ à $C_4$, un radical acide aminé, un résidu d’un dipeptide ou d’un tripeptide,
- $R^3$ et $R^4$ représentent indépendamment l’un de l’autre un atome d’hydrogène, un radical alkyle en $C_1$ à $C_4$ ou un des deux radicaux représente un groupe hydroxy et
- n représente un nombre entier de 1 à 3.

2. Utilisation d’au moins un composé de formule générale (I) ou (II) selon la revendication 1 dans des agents de nettoyage et/ou de traitement des cheveux pour la régulation de la teneur en humidité des cheveux.

3. Utilisation d’au moins un composé de formule générale (1) ou (II) selon la revendication 1 dans des agents de nettoyage et/ou de traitement des cheveux pour augmenter la résistance des cheveux.

4. Utilisation d’au moins un composé de formule générale (I) ou (II) selon la revendication 1 dans des agents de

nettoyage et/ou de traitement des cheveux pour augmenter la brillance des cheveux.

**5.** Utilisation d'au moins un composé de formule générale (I) ou (II) selon la revendication 1 dans des agents de nettoyage et/ou de traitement des cheveux pour diminuer les cassures et les fourches des cheveux.

**6.** Utilisation selon l'une quelconque des revendications 1 à 5, **caractérisée en ce que** le radical $R^1$ représente un groupe méthyle.

**7.** Utilisation selon l'une quelconque des revendications 1 à 6, **caractérisée en ce que** le radical $R^2$ représente le groupement -COOH.

**8.** Utilisation selon l'une quelconque des revendications 1 à 7, **caractérisée en ce que** les radicaux $R^3$ et $R^4$ représentent hydrogène.

**9.** Utilisation selon l'une quelconque des revendications 1 à 8, **caractérisée en ce que** n représente le nombre entier 2.

**10.** Utilisation selon l'une quelconque des revendications 1 à 9, **caractérisée en ce que** le composé selon la formule (I) ou (II) est l'ectoïne ou un de ses sels physiologiquement acceptables.

**11.** Utilisation selon l'une quelconque des revendications 1 à 10, **caractérisée en ce que** l'ectoïne ou ses sels physiologiquement acceptables est utilisé dans les agents de nettoyage et/ou de traitement des cheveux en une concentration de 0,01 à 2% en poids.

**12.** Utilisation selon l'une quelconque des revendications 1 à 11, **caractérisée en ce que** les composés selon la formule générale (I) ou (II) sont utilisés dans les shampooings, les après-shampooings, les produits de traitement pour cheveux, les toniques pour cheveux, les solutions de fixateur pour permanente, les shampooings colorants pour cheveux, les fixateurs pour cheveux, les agents de mise en pli pour cheveux et les compositions de coiffage pour cheveux.